Europäisches Patentamt

European Patent Office

Office européen des brevets

(11) Publication number: **0 328 404 B1**

## EUROPEAN PATENT SPECIFICATION

(45) Date of publication of patent specification: 29.09.93 (51) Int. Cl.⁵: **A61K 39/395**, C12N 15/00

(21) Application number: 89301291.4

(22) Date of filing: 10.02.89

(54) Modified antibodies.

(30) Priority: 12.02.88 GB 8803228
25.02.88 GB 8804464

(43) Date of publication of application:
16.08.89 Bulletin 89/33

(45) Publication of the grant of the patent:
29.09.93 Bulletin 93/39

(84) Designated Contracting States:
AT BE CH DE ES FR GB GR IT LI LU NL SE

(56) References cited:
EP-A- 0 239 400

JOURNAL OF IMMUNOLOGICAL METHODS, vol. 103, 1987, pages 59-67, Elsevier Science Publishers B.V. (Biomedical Division); G. HALE et al.: "Isolation of low-frequency class-switch variants from rat hybrid myelomas"

NATURE, vol. 321, 29th May 1986, pages 522-525; P.T. JONES et al.: "Replacing the complementarity-determining regions in a human antibody with those from a mouse"

NATURE, vol. 332, 24th March 1988, pages 323-327; L. RIECHMANN et al.: "Reshaping

human antibodies for therapy"

(73) Proprietor: **BRITISH TECHNOLOGY GROUP LIMITED**
**101 Newington Causeway**
**London SE1 6BU(GB)**

(72) Inventor: **Clark, Michael Ronald**
**108 York Street**
**Cambridge, CB1 2PY(GB)**
Inventor: **Riechmann, Lutz**
**7064 Vista del Mar Avenue**
**La Jolla California 92037(US)**
Inventor: **Waldmann, Herman**
**11 Gurney Way**
**Cambridge(GB)**
Inventor: **Winter, Gregory Paul**
**64 Cavendish Avenue**
**Cambridge(GB)**

(74) Representative: **Stephenson, Gerald Frederick et al**
**Patents Department British Technology Group Ltd 101 Newington Causeway**
**London SE1 6BU (GB)**

Rank Xerox (UK) Business Services
(3.10/3.6/3.3.1)

**Description**

This invention relates to reshaped antibodies suitable for use in the treatment of human beings or animals.

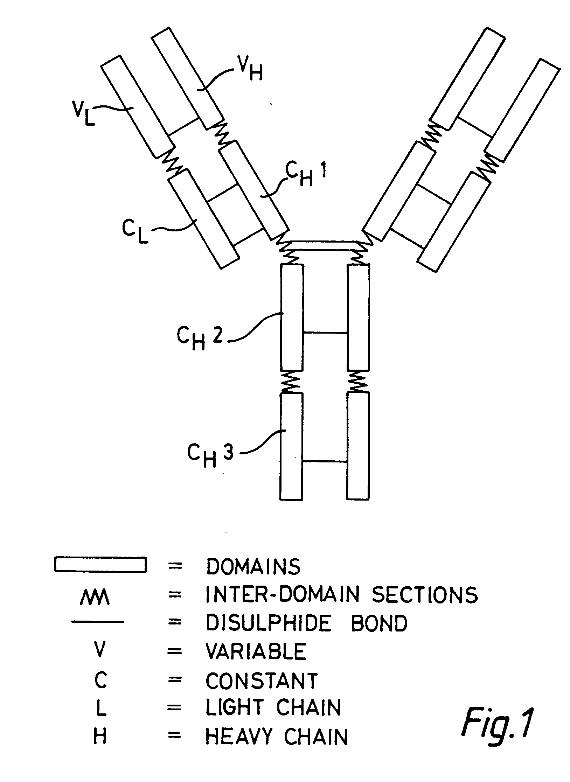

Antibodies, or immunoglobulins, comprise two heavy chains linked together by disulphide bonds and two light chains, each light chain being linked to a respective heavy chain by disulphide bonds. The general structure of an antibody of class IgG (i.e. an immunoglobulin (Ig) of class gamma (G)) is shown schematically in Figure 1 of the accompanying drawings.

Each heavy chain has at one end a variable domain followed by a number of constant domains. Each light chain has a variable domain at one end and a constant domain at its other end, the light chain variable domain being aligned with the variable domain of the heavy chain and the light chain constant domain being aligned with the first constant domain of the heavy chain. The constant domains in the light and heavy chains are not involved directly in binding the antibody to antigen.

The variable domains of each pair of light and heavy chains form the antigen binding site. The domains on the light and heavy chains have the same general structure and each domain comprises four framework regions, whose sequences are relatively conserved, connected by three complementarity determining regions (herein after in the description referred to as CDRs) (see reference 11). The four framework regions largely adopt a beta-sheet conformation and the CDRs form loops connecting, and in some cases forming part of, the beta-sheet structure.

The CDRs are held in close proximity by the framework regions and, with the CDRs from the other domain, contribute to the formation of the antigen binding site.

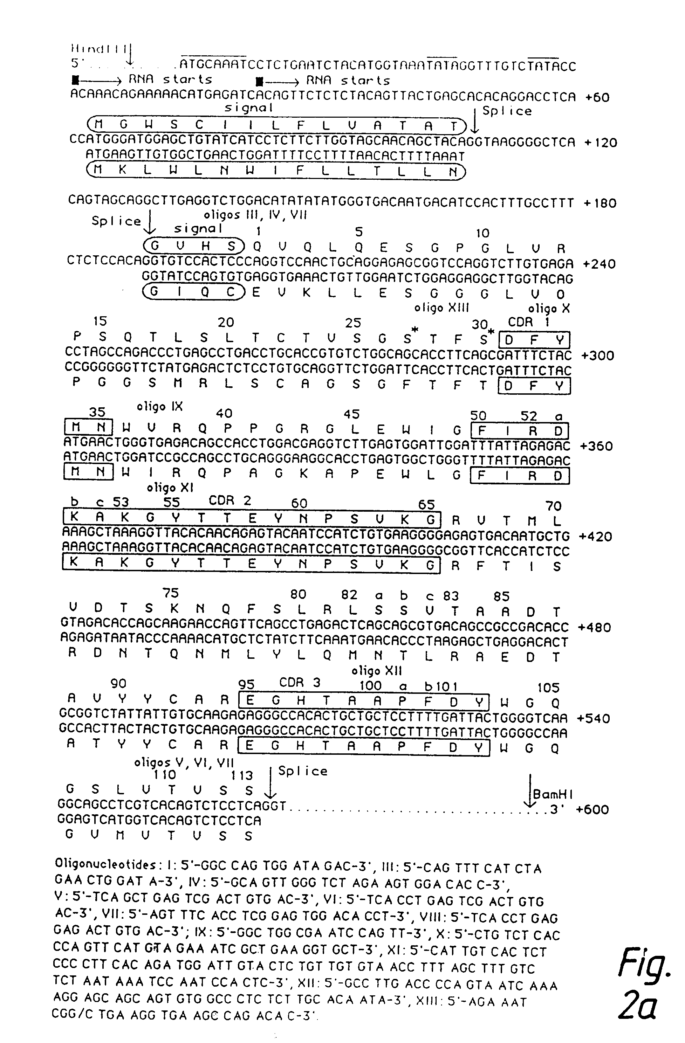

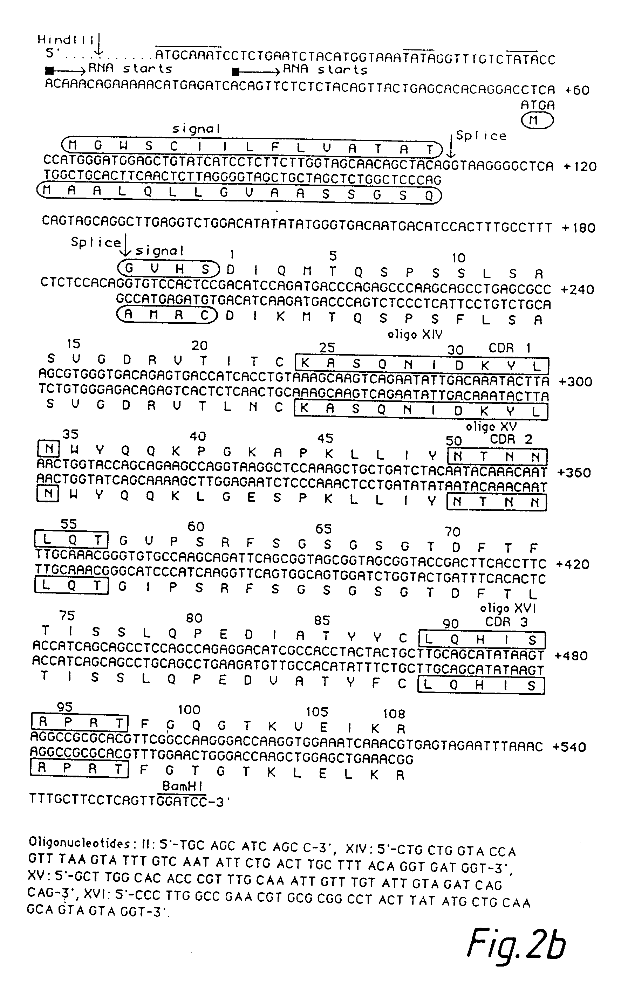

According to the present invention an antibody with affinity for the antigen Campath-1 has at least one CDR which is foreign with respect to the constant region of the antibody, said at least one foreign CDR being selected from CDRs which are substantially as identified in Figure 2, that is residues 31 to 35, 50 to 65 and 95 to 102 of the heavy chain as shown in Figure 2a and residues 24 to 34, 50 to 56 and 89 to 97 of the light chain as shown in Figure 2b, the antibody being capable of binding effectively to the antigen Campath-1.

The term "foreign" is used in relation to the CDR(s) and constant region, i.e. the heavy and light chain constant domains, to mean of different origin.

In Figure 2 and elsewhere in the specification amino acid residues are identified by the conventionally used one letter symbols, as follows:

| Amino Acid | One-letter symbol |
|---|---|
| Alanine | A |
| Arginine | R |
| Asparagine | N |
| Aspartic acid | D |
| Asparagine or aspartic acid | B |
| Cysteine | C |
| Glutamine | Q |
| Glutamic acid | E |
| Glutamine or glutamic acid | Z |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

In this specification, effective antibody-antigen binding indicates that the antibody effects 50% binding to antigen at antibody concentrations of less than or equal to 70 $\mu$g/ml, preferably at concentrations of less than or equal to 7 $\mu$g/ml. Binding affinity may be tested by assay procedures such as are described in Example 1 herein, eg using Campath-1 antigen obtained from a glycolipid extract From human spleen.

Thus, a standard procedure for the extraction of glycolipids can be applied to the extraction of the Campath-1 antigen from human spleens. This standard extraction procedure involves the treatment of 1 volume of honogenised human spleen tissue with 3 volumes of water, 11 volumes of methanol and 5.4 volumes of chloroform. After mixing, precipitated material is discarded and a further 3.5 volumes of water are added, followed by further mixing. The mixture is then allowed to separate into two phases, the lower chloroform-containing phase is discarded and the upper aqueous phase is concentrated to provide a crude extract of the Campath-1 antigen, which can if desired be purified further by affinity chromatography, for example using the YTH66.9 antibody referred to hereinafter.

The antibody of the present invention desirably has a light chain with at least one CDR selected from CDRs substantially as identified in Figure 2 and a heavy chain with at least one CDR selected from CDRs substantially as identified in Figure 2.

As a further possibility, the antibody of the present invention preferably has three heavy chain CDRs substantially as identified in Figure 2, or three light chain CDRs substantially as identified in Figure 2. More preferably, the antibody has all six heavy and light chain CDRs substantially as identified in Figure 2.

Hence, in a preferred aspect of the present invention an antibody with affinity for the antigen Campath-1 has heavy and light chain CDRs which are foreign with respect to tie constant region of the antibody, said CDRs being substantially as identified in Figure 2, that is residues 31 to 35, 50 to 65 and 95 to 102 of the heavy chain as shown in Figure 2a and residues 24 to 34, 50 to 56 and 89 to 97 of the light chain as shown in Figure 2b, the antibody being capable of binding effectively to the antigen Campath-1.

The CDRs identified in Figure 2 are of rat origin and may be combined with a range of different variable domain framework regions, as desired, including, eg, framework regions of rat or human origin.

In a further aspect of the present invention an antibody with affinity for the antigen Campath-1 has heavy and light chain variable domains as identified in the lower lines of sequence information in Figure 2, that is residues 1 to 113 of the heavy chain and residues 1 to 108 of the light chain, the CDRs and constant region of the antibody being foreign with respect to one another, the antibody being capable of binding effectively to the antigen Campath-1.

Such an antibody comprises CDRs and framework regions of rat origin.

The invention also includes an antibody with affinity for the antigen Campath-1 which has heavy and light chain variable domains as identified in the upper lines of sequence information in Figure 2, that is residues 1 to 113 of the heavy chain and residues 1 to 108 of the light chain, and that will bind effectively to the antigen Campath-1. Such an antibody comprises CDRs of rat origin in framework regions of human origin.

Although still being of human origin, framework regions may nevertheless be modified from those which occur in the human. Thus, an antibody having heavy and light chain variable domains as identified in the upper lines of sequence information in Figure 2 may be modified by having a phenylalanine group at residue 27 of the heavy chain in place of serine, and possibly also by having a threonine group at residue 30 of the heavy chain in place of serine. A Ser(27) to Phe mutation is Found to increase antibody-antigen binding significantly. However, the mutation of Ser (30) to Thr (in the human framework with the Ser (27) to Phe mutation) has little effect on binding affinity. This illustrated that point mutations in the antibody may have a major effect or little effect on the affinity of the antibody for the antigen. Although the two changes Ser (27) to Phe and Ser (30) to Thr are located within the framework region as defined in reference 11, they lie within the hypervariable loop H1 as defined in reference 18. It is accordingly believed that some some changes in the CDRs may similarly be made without necessarily having an adverse effect on antibody-antigen affinity. References to CDRs substantially as identified in Figure 2 are accordingly intended to include within their scope not only CDRs identical to those identified in Figure 2 but also variants of such CDRs, subject to the requirement of the antibody binding effectively to Campath-1.

The antibody is preferably in biologically pure form, desirably being at least 95% (by wt) free of other biological materials.

The remainder of the antibody, namely the heavy and light chain constant domains and possibly also variable domain framework regions and one or more CDRs, can be based on antibodies of various different types as desired including, eg, rat and human antibodies of different classes. Thus, the constant domains can be selected to have desired effector functions appropriate to the intended use of the antibody. For example, for therapeutic purposes, human IgG1 and rat IgG2b are currently favoured isotypes. Further, of the human IgG isotypes, IgG1 and IgG3 appear to be the most effective for complement and cell mediated

3

lysis, and therefore for killing tumour cells. For other purposes other isotypes may be favoured, eg, rat IgM, IgG1, IgG2a, IgG2c, human IgG2, IgG4 etc. For human therapy it is particularly desirable to use human isotypes, to minimise antiglobulin responses during therapy.

The Campath-1 antigen is strongly expressed on virtually all human lymphocytes and monocytes, but is absent from other blood cells including the hemopoietic stem cells, the antigen being described by Hale et al in Blood, 1983, 62, 873-882 (reference 6). That paper describes the antibody YTH66.9 which is specific for the Campath-1 antigen, this antibody being available from Serotec of 22 Bankside, Station Approach, Kidlington, Oxford, England, under the designation YTH 66.9 HL. A further series of antibodies to Campath-1 has been produced, including rat monoclonal antibodies of IgM, IgG2a, and IgG2c isotypes (reference 7) and more recently IgG1 and IgG2b isotypes have been isolated as class switch variants from the IgG2a secreting cell line YTH 34.5HL (reference 8). All of these antibodies with the exception of the rat IgG2c isotype are able efficiently to lyse human lymphocytes with human complement.

In addition, the IgG2b antibody YTH 34.5HL-G2b, but not the other isotypes, is effective in antibody dependent cell mediated cytotoxicity (ADCC) with human effector cells (reference 8). These rat monoclonal antibodies have found important application in the context of immunosuppression, for control of graft-versus-host disease in bone narrow transplantation (reference 6); the management of organ rejection (reference 9); the prevention of marrow rejection and in the treatment of various lymphoid malignancies (reference 10). For in-vivo use, the IgG2b antibody YTH 34. 5HL-G2b seems to be the most effective at depleting lymphocytes, but the use of any of the antibodies in this group is limited by the antiglobulin response which can occur within two weeks of the initiation of treatment (reference 10).

Antibodies in accordance with the invention, particularly those based on human isotypes, thus have good therapeutic potential. In particular, the availability of a reshaped human antibody with specificity for the Campath-1 antigen should permit a full analysis of the in vivo potency and immunogenicity of an anti-lymphocyte antibody with wide therapeutic potential. Such reshaped antibodies have been used in the treatment of patients with non-Hodgkin lymphoma, as well as in the treatment of some cases of autoimmune disease. Further trials with organ graft patients, particularly kidney graft patients, are proposed. Even if anti-idiotypic responses are eventually observed, considerable therapeutic benefit could be derived by an extended course of treatment. In addition an antiglobulin response restricted to idiotype should be circumvented by using a series of antibodies with different idiotype (reference 20). In principle, the idiotype of the reshaped Campath-1 could be changed by altering the hypervariable regions or the framework regions: evidence From a reshaped antibody specific for the hapten nitrophenyl acetate suggests that the recognition by anti-idiotypic antisera and anti-idiotypic monoclonal antibodies is influenced by residues in the framework region (reference 5). Thus recycling the hypervariable regions on different human framework regions should change the idiotype, although ultimately it could focus the response directly onto the binding site for Campath-1 antigen. Although such focussing would be undesirable for Campath-1 antibodies, it could be an advantage for the development of anti-idiotypic vaccines.

In a further aspect, the invention thus includes the use of an antibody or a fragment thereof as described herein in the manufacture of a medicament for use in treating patients, particularly humans, with cancers, particularly lymphomas, or for immunosuppression purposes.

Antibodies in accordance with the present invention may be formulated for administration to patients by mixing the antibody purified in conventional manner with a physiologically acceptable diluent or carrier, possibly in admixture with other therapeutic agents such as other antibodies. In one example, purified antibody was reconstituted in a commercially available human plasma protein solution (minus gamma globulin). The formulation was administered by intravenous infusion at the rate of 5 mg antibody per day for at least 10 days.

Antibodies in accordance with the invention can be produced in various different ways, as will be described in greater detail in the following Examples.

Heavy and light chain variable domains are conveniently produced separately and assembled with the remainder of an antibody of desired origin, eg desired human isotype.

Genes encoding the variable domains of an antibody of desired structure may be produced, and attached to genes encoding the constant domains of of an antibody of desired isotype. Genes encoding variable domains can be derived from hybridoma cDNA or from the chromosome. Genes encoding the variable domains are also conveniently constructed by gene synthesis techniques or by site directed mutagenesis using long synthetic oligonucleotides. Expression is conveniently effected by transforming a cell line, eg an immortalised mammalian cell line such as a myeloma cell line, with expression vectors including DNA coding for the variable domains and for the remainder of the antibody and culturing the transformed cell line to produce the desired antibody.

In another aspect of the invention a process for the preparation of an an antibody with affinity for the antigen Campath-1 having at least one CDR which is foreign with respect of the constant region of the antibody, said at least one foreign CDR being selected from CDRs substantially as identified in Figure 2, that is amino acid residues 31 to 35, 50 to 65 and 95 to 102 of the heavy chain as shown in Figure 2a, and amino acid residues 24 to 34, 50 to 56 and 89 to 97 of the light chain as shown in Figure 2b, the antibody being capable of binding effectively to the antigen Campath-1, comprises culturing cells capable of expressing the antibody in order to effect expression thereof.

It will be appreciated that instead of the whole antibody a fragment thereof may be used which retains the CDRs but lacks other parts of the whole molecule not essential to its binding efficacy, particularly a F-(ab')₂ fragment, and such fragments retaining the binding capability of the antibody are included by the present invention.

The invention will be further described, by way of illustration, in the following Examples which refer to the accompanying drawings, in which:

Figure 1 is a schematic diagram illustrating the structure of an IgG molecule:

Figure 2 illustrates nucleic acid and amino acid sequences of the variable domains of antibodies in accordance with the invention, with Figure 2a representing the heavy chain and Figure 2b representing the light chain. The upper line of the Figure gives sequence information for the reshaped antibody containing framework regions of human origin, with the lower line giving sequence information for rat YTH 34.5HL antibody;

Figure 3 illustrates the sequence of the HuVLLYS° gene and derived amino acid sequence;

Figure 4 illustrates the sequence of the HuVLLYS gene and derived amino acid sequence;

Figure 5 illustrates a strategy for producing a reshaped human antibody having rat CDRs;

Figure 6 illustrate loop Phe 27 to Tyr 35 in the heavy chain variable domain of the human myeloma protein KOL;

Figure 7 illustrates the results of complement lysis and ADCC for various antibodies;

Figure 8 illustrates the results of complement lysis and ADCC of various further antibodies;

Figure 9 shows the effect of CAMPATH-1H on blood counts in a patient (patient 1), with solid triangles showing results for lymphocytes and empty triangles results for neutrophils;

Figure 10 shows the cytology of bone marrow cells from two (patients 1 and 2) patients treated with CAMPATH-1H:

A = patient 1 trephine before treatment with CAMPATH-1H
B = patient 1 trephine on day 43 (ie 16 days after treatment)
C = patient 2 aspirate before treatment with CAMPATH-1H
D = patient 2 aspirate on day 78 (ie 35 days after treatment);

Figure 11 shows computed tomography scans from patients 1 and 2, showing affected spleens and lymphonode:

A = patient 1 before treatment with CAMPATH-1H
B = patient 1 on day 57
C = patient 2 before treatment with CAMPATH-1H (retrocrural node arrowed)
D = patient 2 on day 51; and

Figure 12 shows the effect of CAMPATH-1H on blood counts in patient 2, with solid triangles showing results for lymphocytes and empty triangles results for neutrophils.

Example 1

The sequences of the heavy and light chain variable domains of rat IgG2a Campath-1 antibody YTH 34.5HL were determined by cloning the cDNA (Figure 2), and the hypervariable regions were identified according to Kabat (see reference 11). Sequence information is given in the lower lines of Figure 2, with the CDRs identified in boxes.

In the heavy chain variable domain there is an unusual feature in the framework region. In most known heavy chan sequences Pro(41) and Leu(45) are highly conserved: Pro(41) helps turn a loop distant from the antigen binding site and Leu(45) is in the beta bulge which forms part of the conserved packing between heavy and light chain variable domains (reference 12). In YTH 34.5HL these residues are replaced by Ala-(41) and Pro(45), and presumably this could have some effect on the packing of the heavy and light chain variable domains.

Working at the level of the gene and using three large mutagenic oligonucleotides for each variable domain, in a single step the hypervariable regions of YTH 34.5HL were mounted on human heavy or light chain framework regions taken from the crystallographically solved proteins NEW for the heavy chain (reference 13) and from a protein based closely on the human myeloma protein REI for the light chain (reference 14). The NEW light chain was not used because there is a deletion at the beginning of the third framework region of the NEW light chain. The resulting reshaped heavy chain variable domain HuVHCAMP is based on the HuVHNP gene (references 1, 5) with the framework regions of human NEW alternating with the hypervariable regions of rat YTH 34.5HL. There are discrepancies involving the first framework region and the first hypervariable loop of the NEW heavy chain between the published sequence used here and the sequence deposited in the Brookhaven data base (in parentheses): Ser27 (to Thr), Thr28 (to Ser) and Ser30 (to Asp). Neither version is definitive and the discrepancies do not affect present considerations. The reshaped light chain variable domain HuVLCAMP is a similar construct, except with essentially the framework regions of the human myeloma protein REI, with the C-terminal and the 3′ non-coding sequence taken from a human $J_k$-region sequence (reference 22). Sequence information for the variable domain of the reshaped antibody is given in the upper lines in Figure 2. The sequences of oligonucleotide primers are given and their locations on the genes are also marked in Figure 2.

Considering the above in further detail, mRNA was purified (reference 23) from the hybridoma clone YTH 34.5HL (gamma 2a, $k^b$), and first strand cDNA made by priming with oligonucleotides complementary to the 5′ end of the CH1 (oligonucleotide I) and the Ck exons (oligonucleotide II). cDNA was cloned and sequenced as described in references 24 and 25.

For expression of the rat heavy chain variable domain RaVHCAMP, two restriction sites (XbaI and SalI) were introduced at each end of the cDNA clone in M13 using mutagenic oligonucleotides III and V respectively, and the XbaI-SalI fragment excised. Simultaneously, the corresponding sites were introduced into the M13-HuVHNP gene using oligonucleotides IV and VI, and the region between the sites exchanged. The sequence at the junctions was corrected with oligonucleotides VII and VIII, and an internal BamHI site removed using the oligonucleotide IX, to create the M13-RaVHCAMP gene. The encoded sequence of the mature domain is thus identical to that of YTH 34.5HL.

The reshaped heavy chain variable domain (HuVHCAMP) was constructed in an M13 vector by priming with three long oligonucleotides simultaneously on the single strand containing the M13-HuVHNP gene (references 1, 5). The mutagenesis techniques used were similar to those described in reference 33, using the host 71-18 mutL and without imposing strand selection. Each oligonucleotide (X, XI and XII) was designed to replace each of the hypervariable regions with the corresponding region from the heavy chain of the YTH 34.5HL antibody.

Colony blots were probed initially with the oligonucleotide X and hybridisation positives were sequenced: the overall yield of the triple mutant was 5%. Ser27 to Phe and Ser27 to Phe, Ser30 to Thr mutants (to be described below) of M13mp8-HuVHCAMP were made with the mixed oligonucleotide XIII.

The reshaped light chain variable domain (HuVLCAMP) was constructed in an M13 vector from a gene with framework regions based on human REI. As above, three long oligonucleotides (XIV, XV, and XVI) were used to introduce the hypervariable regions of the YTH 34.5HL light chain.

Construction of the humanised light chain variable domain is described in greater detail in the following seven paragraphs.

(1) The "humanised" light chain variable domain (HuVLCAMP) was constructed in three stages, utilising a "humanised" light chain variable domain (HuVLLYS) which had been constructed for other purposes.

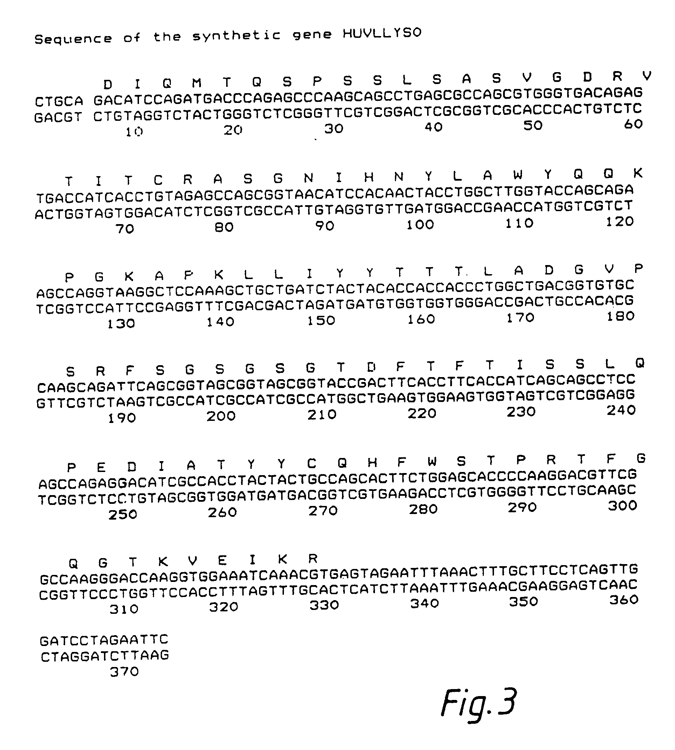

(a) The first stage involved the gene synthesis of a "humanised" light chain variable domain gene (HuVLLYS°). The HuVLLYS° gene incorporates human framework regions identical to the most common residue in each position in the Kabat alignment of the human kappa subgroup I, except for residues 97-108, which were identical to those in the human J1 fragment described in reference 34. The sequences of the framework regions are very similar to the crystallographically solved light chain structure REI. The CDRs in HuVLLY°s were identical to those in the mouse antilysozyme antibody (Dl.3) light chain (unpublished). A 30 bp sequence, identical to the sequence following the genomic Jl segment, was introduced to the 3′ side of residue 108. BamH1 and EcoRI restriction sites were introduced at the 3′ end of the synthetic gene, and a PstI site at the 5′ end. The gene synthesis of HuVLLYS° is described in paragraphs (2) to (5) below, and the sequence of the gene and the derived amino acid sequence is given in Figure 3.

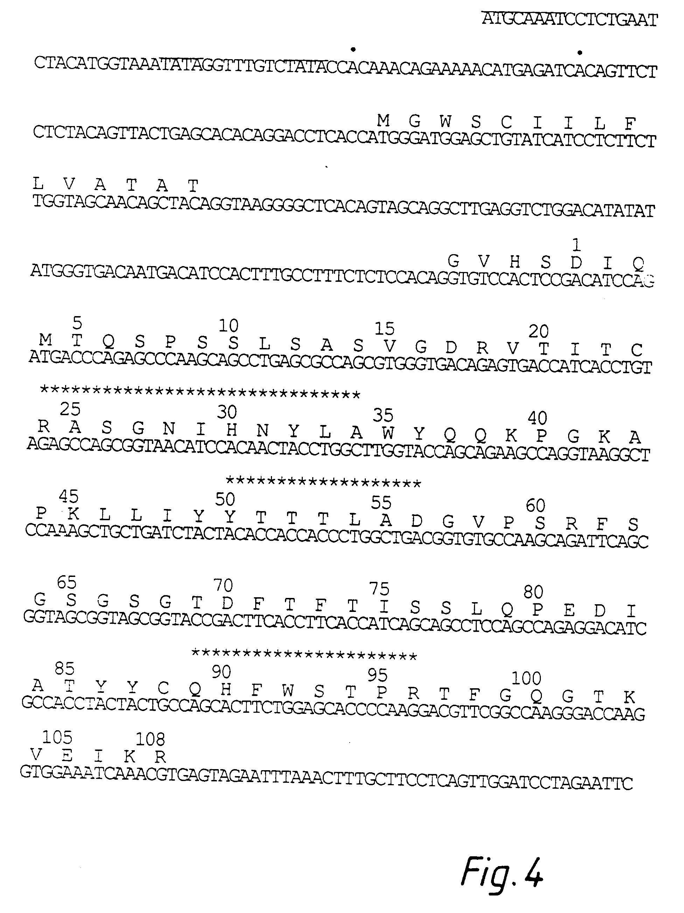

(b) The second stage involved the introduction of the HuVLLYS° gene in place of the heavy chain variable domain in the vector M13-MOVHNP and this is described in pargraphs 6 and 7 below. Thus the light chain variable domain utilises the promoter and signal sequence of a heavy chain variable domain: at the 3′ end of the gene the sequence is derived from the human light chain J1 segment as described in paragraph (1a). The sequence of the HuVLLYS gene and the derived amino acid sequence is given in Figure 4.

(c) The third stage involved the conversion of HuVLLYS to a "humanised" light chain variable domain with the CDRs of Campath-1 specificity.

2. For the synthesis of the HuVLLYS° gene, three blocks of oligonucleotides (PK1-5, KK1-5 and KE1-8 in the table in paragraph 3 below were cloned separately into M13 vectors, and sequenced. Each cloned block was excised and ligated together into M13mp19 to create the HuVLLYS° gene.

3. Oligonucleotides listed below were produced on an Applied Biosystems 380B synthesizer. Each oligonucleotide was size-purified, 10 nmol being subjected to electrophoresis on a 20 x 40 cm 12% polyacrylamide, 7M urea gel, eluted from the gel by dialysis against water, and lyophilized. For gene synthesis or mutagenesis, a 50 pmol aliquot of each purified oligonucleotide was phosphorylated in a 20 $\mu$l reaction mixture with 50mM Tris-Cl (pH 8.0), 10mM $MgCl_2$, 5mM dithiothreitol, 1 mM ATP, and 5 units of polynucleotide kinase, incubated at 37° for 30 minutes. When used as hybridization probes, gel-purified oligonucleotides were phosphorylated in a similar fashion, except on a 15 pmol scale with an excess of $^{32}P$ labeled ATP.

```
name        sequence (5'-3')


PK1         GACATCCAGATGACCCAGAGCCCAAGCAGCCTGAGCGCCAGCGTGGGT
PK2         GACAGAGTGACCATCACCTGTAGAGCCAGCGGTAACATCCACAACTAC
            CTGGCTTGGTAC
PK3         CAAGCCAGGTAGTTGTGGATGTTACCGCTGGCTCTACAGGTGAT
PK4         GGTCACTCTGTCACCCACGCTGGCGCTCAGGCT
PK5         GCTTGGGCTCTGGGTCATCTGGATGTCTGCA


KK1         CAGCAGAAGCCAGGTAAGGCTCCAAAGCTGCTGATCTACTACACCACC
            A
KK2         CCCTGGCTGACGGTGTGCCAAGCAGATTCAGCGGTAGCGGTAGCGGTA
            C
KK3         CGCTACCGCTACCGCTGAATCTGCT
KK4         TGGCACACCGTCAGCCAGGGTGGTGGTGTAGTAGATCAGC
KK5         AGCTTTGGAGCCTTACCTGGCTTCTGCTGGTAC


KE1         CGACTTCACCTTCACCATCAGCAGCCTCCAGCCAGAGGACATCGCCAC
            CTACTACTGCC
KE2         AGCACTTCTGGAGCACCCCAAGGACGTTCGGCCAAGGGACCAAGGTGG
            A
KE3         AATCAAACGTGAGTAGAATTTAAACTTTGCTTCCTCAGTTGGATCCTA
            G
```

KE4      AATTCTAGGATCCAACTGAGGAAGCAAAGTTTAAA

KE5      TTCTACTCACGTTTGATTTCCACCTTGGTCCCTT

KE6      GGCCGAACGTCCTTGGGGTGCTCCAGAAGTGCTGGCAGTAGTAG

KE7      GTGGCGATGTCCTCTGGCTGGAGGCT

KE8      GCTGATGGTGAAGGTGAAGTCGGTAC

PK0      TCATCTGGATGTCGGAGTGGACACCT

4. The construction of individual blocks is described for the PK1-5 block, but KK1-5 and KE1-8 blocks were cloned as KpnI-KpnI and KpnI-EcoRI fragments respectively in a similar way. 4μl portions of each oligonucleotide PK1, PK2, PK3, PK4 and PK5, kinased as in paragraph 3, were combined and annealed at 80°C for 5 minutes, 67°C for 30 minutes, and allowed to cool to room temperature over the span of 30 minutes, 0.1μl of this annealing mix was ligated with 20 ng of PstI/KpnI digested M13-mp19, in 10μl 50mM Tris-Cl (pH7.5), 10mM MgCl₂, 10mM dithiothreitol, 1 mM ATP, 120 units T4 DNA ligase (Biolabs), and incubated 12 hours at 15°C. The ligation mix was used to transfect competent E. coli strain BMH 71-18, plated with BCIG and IPTG, and a clone containing the complete PstI-KpnI insert was identified.

5. The three cloned blocks were excised from 10μg double-stranded replicative form of the thee M13 vectors, by digestion with PstI/KpnI (block PK1-5), KpnI (block KKI-5) and KpnI/EcoRI (block KE1-8). The inserts were separated from the vector by electrophoresis on a 20 x 20 cm 12% polyacrylamide gel, eluted from the gel slices with 0.5 M NH₄OAc, 10 mM Mg (OAc)₂, 0.1 mM EDTA, 0.1% SDS, and purified by phenol extraction and ethanol precipitation. All three fragments were ligated to PstI/EcoRI cut M13-mp19. 200 white plaques were transferred by toothpick to a fresh 2xTY plate, and grown as a grid of infected colonies. The plate was blotted with nitrocellulose filters, which were then treated with 0.5 M NaOH, followed by 1M Tris-Cl (pH7.5), and baked 1 hr at 80°C under vacuum. The filters were washed at 67°C in 3x Denhardt's solution, 2xSSC, 0.07% SDS, followed by 6xSSC at room temperature. Filters were then probed with the radiolabeled oligonucleotides KK3 or KK4 in 3ml of 6xSSC at 37°. Following hybridization with both olignucleotides, positive colonies were picked for DNA sequencing. A phage clone containing correctly assembled blocks was designated M13-HuVLLYS°.

6. To introduce the HuVLLYS° gene in place of the heavy chain variable domain in the vector M13-MOVHNP (described in reference 5) as MV$_{NP}$ with HindII site at the 3′ end of the reading frame), double-stranded replicative form DNA of phages M13-HuVLLYS° and M13-MOVHNP were prepared and digested with PstI and BamHI. The insert of M13-HuVLLYS was isolated on a polyacrylamide gel, and the vector portion of M13-MOVHNP was isolated on an agarose gel. The purified fragments were ligated and transfected into E. coli strain BMH71-18, and the resulting plaques probed with oligonucleotide KK3 to identify the insert. The clone was designated M13-HuVLLYS*.

7. In the M13-HuVLLYS* gene, to join the signal sequence of MOVHNP correctly to the 5′ end of the HuVLLYS° gene (at the PstI site), single stranded DNA was prepared and altered by oligonucleotide directed mutagenesis with the primer PK0- see paragraph (3) for sequence. The mutant clone was designated M13-HuVLLYS.

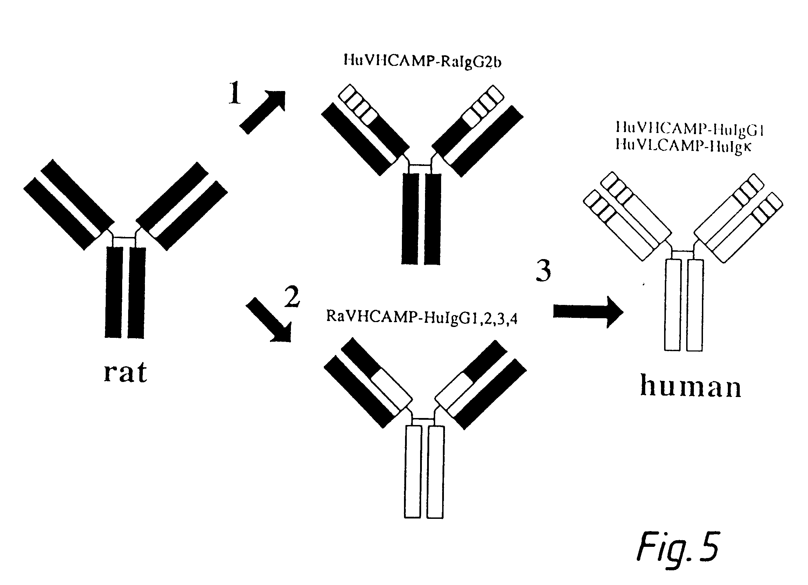

The reshaped human heavy and light chain variable domains were then assembled with constant domains in three stages as illustrated in Figure 5. In Figure 5 sequences of rat origin are marked in black, and those of human origin in white. The recombinant heavy and light chains are also marked using a systematic nomenclature.

The illustrated procedure permits a step-wise check on the reshaping of the heavy chain variable domain (stage 1), the selection of the human isotype (stage 2), and the reshaping of the light chain variable domain and assembly of human antibody (stage 3). The vector constructions were genomic, with the variable domains excised from the M13 vectors and cloned as HindIII-BamHI fragments and the constant domains as BamHI-BamHI fragments in either pSVgpt (heavy chain) (reference 15) or pSVneo (light chain) (reference 16) vectors. The heavy chain enhancer was included to the 5′ side of the variable domain, and expression of both light and heavy chains was driven from heavy chain promoter and the heavy chain signal sequence.

The human gamma 1 (reference 26), gamma 2 (reference 27), gamma 3 (reference 28), gamma 4 (reference 21) and K (reference 22) constant domains, and the rat gamma 2b (reference 29) constant domains were introduced as BamHI-BamHI fragments. The following plasmids were constructed and transfected into lymphoid cell lines by electroporation (reference 30). In stage 1, the pSVgpt vectors HuVHCAMP-RaIgG2B, and also two mutants for reasons to be explained below, HuVHCAMP(Ser27 to Phe)-RaIgG2B, HuVHCAMP(Ser27 to Phe, Ser30 to Thr)-RaIgG2B) were introduced into the heavy chain loss variant of YTH34.5HL. In stage 2, the pSVgpt vectors RaVHCAMP-RaIgG2B, RaVHCAMP-HuIgG1, RaVHCAMP-HuIgG2, RaVHCAMP-HuIgG3, RaVHCAMP-HuIgG4 were transfected as described above. In stage 3, the pSVgpt vector Hu(Ser27-Phe, Ser30-Thr)VHCAMP-HuIgG1 was cotransfected with the pSV-neo vector HuVLCAMP-HuIgK into the rat myeloma cell line Y0 (Y B2/3.0 Ag 20) (ref. 17). In each of the three stages, clones resistant to mycophenolic acid were selected and screened for antibody production by ELISA assays. Clones secreting antibody were subcloned by limiting dilution (for Y0) or the soft agar method (for the loss variant) and assayed again before 1 litre growth in roller bottles.

## Heavy chain variable domain

In stage 1, the reshaped heavy chain variable domain (HuVHCAMP) was attached to constant domains of the rat isotype IgG2b and transfected into a heavy chain loss variant of the YTH34.5 hybridoma. The loss variant carries two light chains, one derived from the Y3 fusion partner (reference 17). The cloned rat heavy chain variable domain (RaVHCAMP) was also expressed as above.

Antibodies were harvested at stationary phase and concentrated by precipitation with ammonium sulphate, followed by ion exchange chromatography on a Pharmacia MonoQ column. The yields of antibody were measured by an ELISA assay directed against the rat IgG2b isotype, and each adjusted to the same concentration (reference 21).

The HuVHCAMP and RaVHCAMP antibodies - all of the rat IgG2b isotype - were compared in a direct binding assay to the Campath-1 antigen (obtained from a glycolipid extract from human spleen), and also in complement lysis of human lymphocytes. For measuring the binding to antigen, the partially purified Campath-1 antigen was coated onto microtitre wells. Bound antibody was detected via a biotin labelled anti-rat IgG2b monoclonal antibody (reference 21), developed with a streptavidin-peroxidase conjugate (Amersham plc). Complement lysis of human lymphocytes with human serum as the complement source was as described in reference 7. For both binding and complement assays, the titres for the antibodies were determined by fitting the data to a sigmoid curve by a least squares iterative procedure (reference 7), and the concentration of antibody giving 50% maximal binding or lysis was noted.

The results are given in Table 1.

Table 1

| Reshaping the heavy chain variable domain | | |
|---|---|---|
| | Concentration of antibody in μg/ml at 50% binding or lysis | |
| heavy chain variable domain | antigen binding | complement lysis |
| RaVHCAMP | 0.7 | 2.1 |
| HuVHCAMP | 27.3 | (*) |
| HuVHCAMP (Ser27 to Phe) | 1.8 | 16.3 |
| HuVHCAMP (Ser27 to Phe,Ser30 to Thr) | 2.0 | 17.6 |

(*) Complement lysis with the HuVHCAMP variable domain was too weak for the estimation of lysis titre.

Compared with the original rat antibody, or the engineered equivalent, the antibody with the reshaped heavy chain domain HuVHCAMP bound poorly to the Campath-1 antigen and was weakly lytic. This suggested an error in the design of the reshaped domain.

There are several assumptions underlying the transfer of hypervariable loops from one antibody to another, and in particular that the antigen binds mainly to the hypervariable regions. These are defined as regions of sequence (reference 11) or structural (reference 18) hypervariability, and the locations of hypervariable regions are similar by either criterion, except for the first hypervariable loop of the heavy chain. By sequence the first hypervariable loop extends from residues 31 to 35 (reference 11) and by structure from residues 26 to 32 (reference 18). Residues 29 and 30 to form part of the surface loop, and residue 27 which is phenylalanine or tyrosine in most sequences including YTH34.5HL, helps pack against residues 32 and 34.

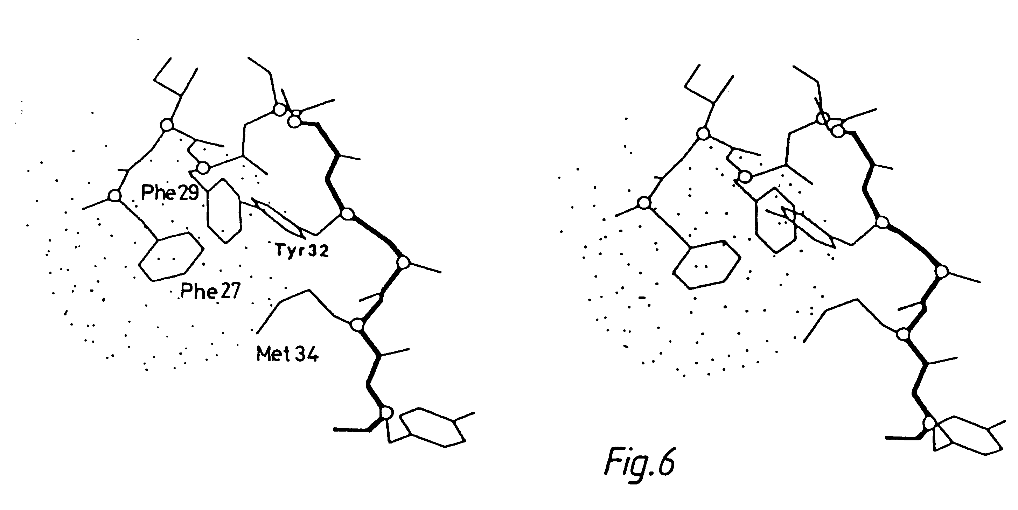

By way of illustration, see Figure 6 which illustrates loop Phe27 to Tyr35 in the heavy chain variable domain of the human myeloma protein KOL which is crystallographically solved (reference 31). The backbone of the hypervariable region according to Kabat (reference 11) is highlighted, and a 200% van der Waal surface is thrown around Phe27 to show the interactions with Tyr32 and Met34 of the Kabat hypervariable region. In the rat YTH34.5HL heavy chain, these three side chains are conserved, but in HuVHCAMP, Phe27 is replaced by Ser: this is because, unlike most human heavy chains, in NEW the phenylalanine is replaced by serine, which would be unable to pack in the same way as phenylalanine. To restore the packing of the loop, a Ser(27) to Phe mutation was made in HuVHCAMP, and also a double mutation Ser(27) to Phe, Ser(30) to Thr (as mentioned above).

The two mutants showed a significant increase in binding to CAMPATH-1 antigen and lysed human lymphocytes with human complement. See the results given in Table 1. Thus the affinity of the reshaped antibody could be restored by altering the packing between the hypervariable regions and the framework by a single Ser(27) to Phe mutation. This suggests that alterations in the "Kabat" framework region can enhance the affinity of the antibody, and extends previous work in which an engineered change in the hypervariable region yielded an antibody with increased affinity (reference 19).

Heavy chain constant domains

In stage 2 (Figure 5), the rat heavy chain variable domain was attached to constant domains of the human isotypes IgG1, 2, 3, and 4, and transfected into the heavy chain loss variant of the YTH34.5 hybridoma.

Antibody was harvested from cells in stationary phase, concentrated by precipitation with ammonium sulphate and desalted into phosphate buffered saline (PBS). Antibodies bound to the Campath-1 antigen coated on microtitre plates, were assayed in ELISA directed against the rat k light chain (reference 21), and adjusted to the same concentration. The antibodies were assayed in complement lysis (as described above) and ADCC with activated human peripheral blood mononuclear cells (references 21, 32). Briefly, $5 \times 10^4$ human peripheral blood cells were labelled with $^{51}Cr$ and incubated for 30 minutes at room temperature with different concentrations of antibody. Excess antibody was removed and a 20 fold excess of activated cells added as effectors. After 4 hours at 37°C death was estimated by $^{51}Cr$ release.

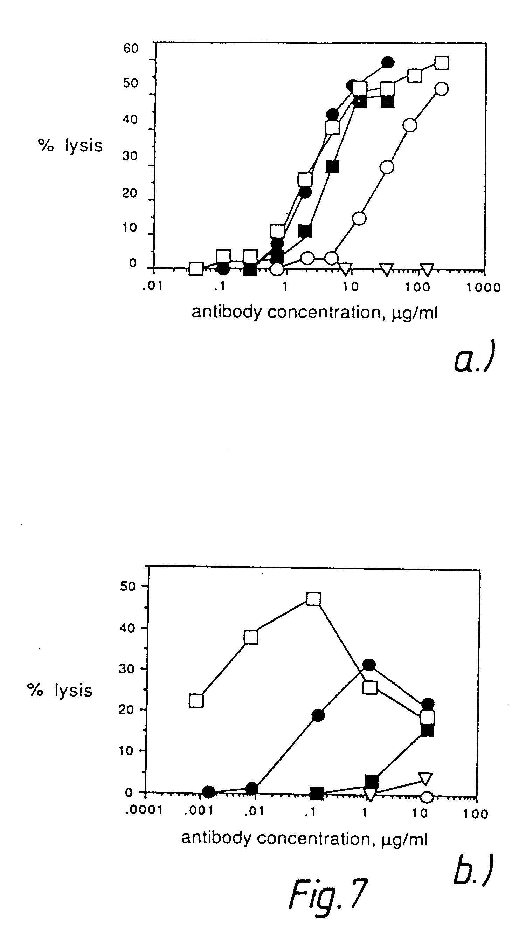

The results are shown in Figure 7, in which the results for rat heavy chain variable domain attached to different human isotypes are represented as follows:

IgG1     empty squares
IgG2     empty circles
IgG3     solid squares
IgG4     empty triangles

Results of lysis with the antibody YTH34.5HL are represented by solid circles.

In complement lysis (Figure 7a), the human IgG1 isotype proved similar to the YTH34.5HL-G2b, with the human IgG3 isotype less effective. The human IgG2 isotype was only weakly lytic and the IgG4 isotype non-lytic. In ADCC (Figure 7b) the human IgG1 was more lytic than the YTH34.5HL-G2b antibody. The decrease in lysis at higher concentration of the rat IgG2b and the human IgG1 antibody is due to an excess of antibody, which causes the lysis of effector cells. The human IgG3 antibody was weakly lytic, and the IgG2 and IgG4 isotypes were non-lytic.

The human IgG1 isotype was therefore suitable for a reshaped antibody for therapeutic use. Other recent work also suggests the IgG1 isotype as favoured for therapeutic application. When the effector functions of human isotypes were compared using a set of chimaeric antibodies with an anti-hapten variable domain, the IgG1 isotype appeared superior to the IgG3 in both complement and cell mediated lysis (reference 4). Furthermore, of two mouse chimaeric antibodies directed against cell surface antigens as tumour cell markers, with human IgG1 or IgG3 isotypes, only the IgG1 isotype mediated complement lysis (references 2, 3).

Light chain

In stage 3 (Figure 5), the reshaped heavy chain was completed, by attaching the reshaped HuVHCAMP (Ser27 to Phe, Ser30 to Thr) domain to the human IgG1 isotype. The reshaped light chain domain HuVHCAMP was attached to the human Ck domain. The two vectors were cotransfected into the non-secreting rat Y0 myeloma line.

Antibody HuVHCAMP (Ser27 to Phe, Thr30 to Ser)-HuIgG1, HuVLCAMP-HuIgK was purified from supernatants of cells in stationary phase by affinity chromatography on protein A Sepharose. The antibody was at least 95% (by wt) pure. The yield (about 10mg/l) was measured spectrophotometrically. Complement and ADCC assays were performed as described in connection with Figure 7.

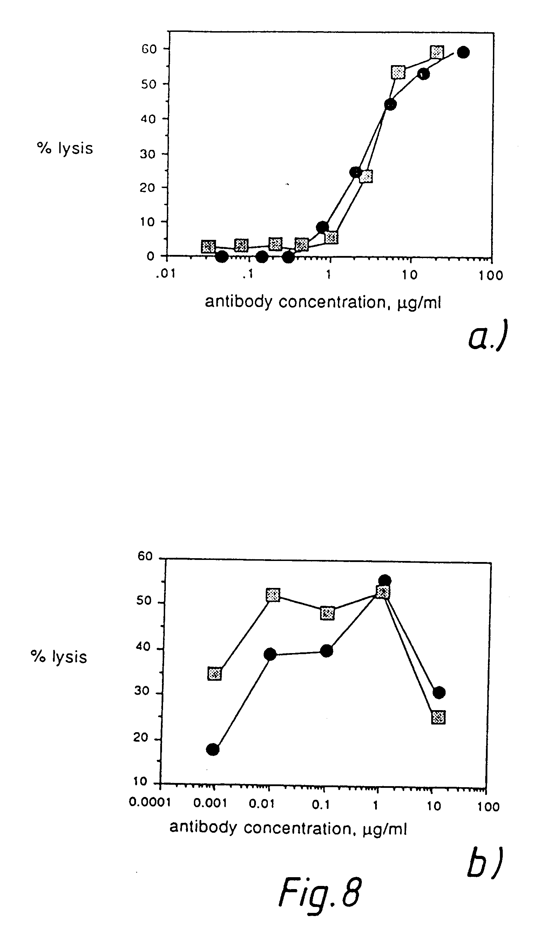

The results are shown in Figure 8, in which the results are reshaped human antibodies are represented by squares and those for rat YTH34.5HL antibodies are represented by solid circles.

The purified antibody proved almost identical to the YTH34.5HL-G2b antibody in complement lysis (Figure 8a). In cell mediated lysis the reshaped human antibody was more reactive than the rat antibody (Figure 8b). Similar results to the ones in Figure 8b were obtained with three different donors of target and effector cells (data not shown). Furthermore the antibody was as effective as YTH34.5HL-G2b in killing leukaemic cells from three patients with B Cell lymphocytic leukaemia by complement mediated lysis with human serum.

The rat antibody and fully humanised antibody were compared in a direct binding assay to Campath-1 antigen. Antibody concentrations were determined as described in Figures 7 and 8. The amount of rat antibody bound to partially purified Campath-1 antigen was determined as described in connection with Table 1. The amount of human antibody bound was determined by an ELISA assay using a biotinylated sheep anti-human IgG antibody (Amersham).

Table 2

| Reshaping the heavy and light chain variable domains simultaneously | |
| --- | --- |
| | Concentration of antibody in $\mu$g/ml at 50% binding |
| antibody | antigen binding |
| RaVHCAMP RaIGG2B<br>RaVHCAMP RaKappa<br>HuVHCAMP (Ser27 to Phe, Ser30 to Thr) HuIGG1<br>HuVLCAMP HuKappa | 1.01<br><br>1.11 |

Thus by transplanting the hypervariable regions from a rodent to a human antibody of the IgG1 subtype, the antibody can be reshaped for therapeutic application.

The strategy illustrated in Figure 5 is stepwise assembly to allow any problems to be detected at each stage (reshaping of heavy chain variable domain, selection of constant domain and reshaping of light chain variable domain). It is quite possible to build the reshaped antibody in a single step assembly, i.e. constructing the two reshaped variable domains, attaching to appropriate constant domains and cotransfecting into e.g. YO.

Example 2

Patients and Methods

Antibody HuVHCAMP (Ser 27 to Phe, Thr 30 to Ser) - HuIgG1, HuVLCAMP - HuIgK, hereinafter referred to as CAMPATH-1H, was prepared as described in Example 1. The CAMPATH-1H antibodies were obtained from culture supernatant of cells growing in a hollow fibre bioreactor ('Acusyst - Jr', Endotronics) and purified by affinity chromatography on protein-A-'sepharose'. They were dissolved in phosphate-buffered saline, sterile filtered, and tested for pyrogen and sterility. Patients were prehydrated over night and antibody, diluted in 500 ml saline, was infused over 2-4 hours.

Campath-1 expression on tumour cells was measured by flow cytometry and complement-mediated lysis (references 6, 35). Serum concentrations of CAMPATH-1H were measured by immunofluorescence with normal lymphocytes. Southern blot analysis with an immunoglobulin $J_H$ probe was used to detect residual tumour cells in DNA extracted from mononuclear fractions of bone marrow. Antiglobulin responses were sought by two techniques. The first was a solid-phase enzyme-linked assay using microlitre plates coated with CAMPATH-1H. After incubation with patients' serum samples, the assay was developed with biotin-labelled CAMPATH-1H followed by streptavidin-peroxidase. A mixture of monoclonal mouse antibodies against human IgG was used as a positive control and 500 ng/ml of this mixture would be detected. In the second assay, patients serum samples were mixed with red cells coupled with CAMPATH-1H (reference 10). Agglutination by 5 ng/ml of the control mixture would be detected. Immunoglobulin allotypes were determined by means of standard reagents and techniques from the Central Laboratory of the Netherlands Red Cross Blood Transfusion Service.

RESULTS

Patient 1

A 69-year-old woman presented in 1983, and grade 1, stage IVA non-Hodgkin lymphoma in leukaemic phase was diagnosed. Between 1983 and 1988 the patient received various types of treatment, including chemotherapy and radiotherapy and rat antibody to Campath-1. She was then given treatment with CAMPATH-1H.

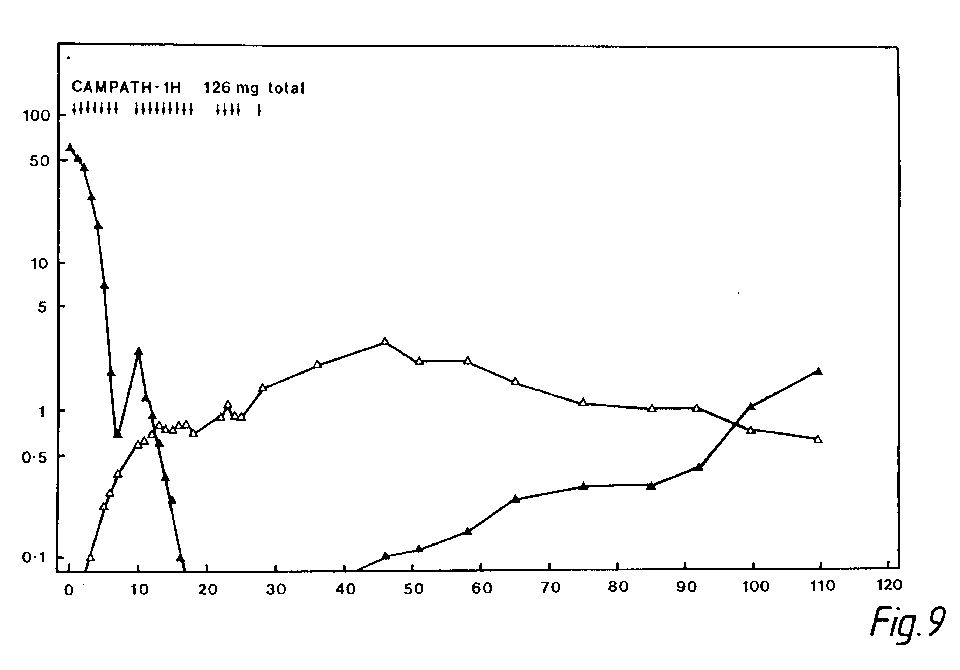

The starting dose was 1 mg daily and, since it was well tolerated, the dose was increased to a maximum of 20 mg/day, though the usual was 4 mg/day owing to the small amount available. In all the patient received 126 mg over 30 days. The response was prompt; in 6 days night sweats had abated, by day 10 there was pronounced reduction in splenomegaly and recovery of blood neutrophils, and by day 18 lymphoma cells were cleared from the blood (Figure 9).

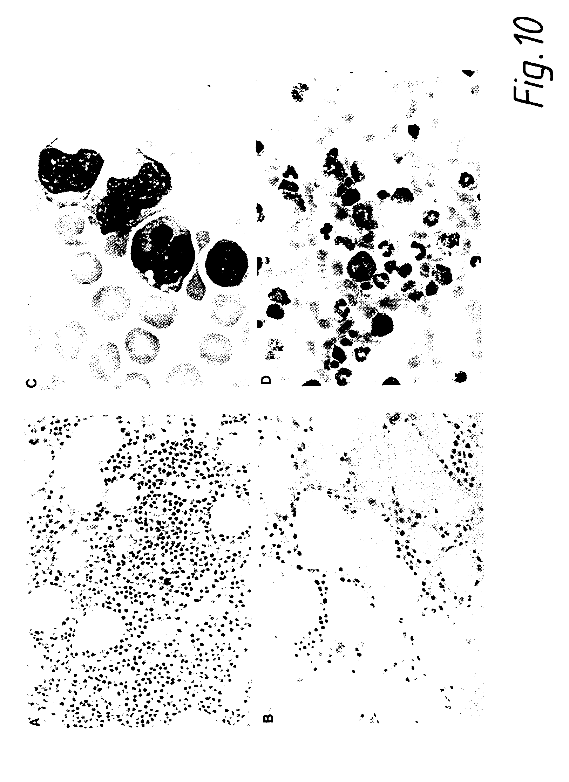

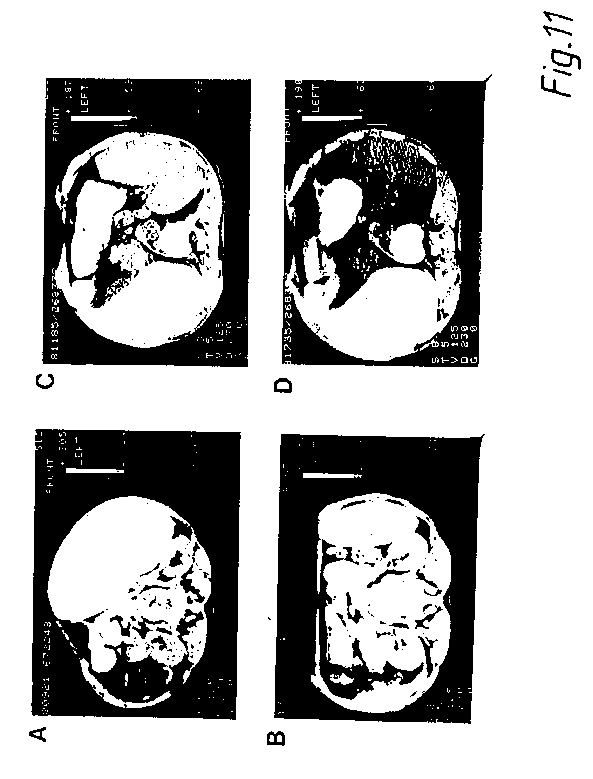

On day 28 a bone marrow aspirate and trephine were hypocellular but showed active myelopoiesis and erythropoiesis and no lymphoid cells (Figure 10B). No CAMPATH-1 positive cells could be detected by flow cytometry. DNA from the mononuclear marrow cells was germline when probed with an immunogloublin $J_H$ probe under conditions where clonal rearrangements could be detected in 0.2% of cells. Thus, it is concluded that lymphoma cells were cleared from the marrow. The spleen volume was reduced about eight-fold (Figures 11A, B), although it was still slightly larger than normal.

Other than fever occurring about 1 hour after the end of antibody infusions there were no adverse effects of antibody treatment until the 5th week, when severe rigors occurred after each infusion. No antiglobulin response could be detected and the rate of clearance of antibody from the serum was unchanged. For the next 3 weeks the patient continued to experience occasional fever and rigors. She was given oral cotrimoxazole because of her lymphopenia, but no infective cause of these symptoms could be found.

In the next 4 months lymphocytes, which appeared morphologically normal, slowly reappeared in the blood (up to $0.2 \times 10^9$/l). They did not show the characteristic rearranged immunogloublin fragments, and both CD3-positive and CD19-positive cells were present (see Table 3). Serum immunoglobulin levels, which had been very low since presentation, have risen towards normal (Table 3). A marrow aspirate and trephine taken 50 days after the end of treatment were again hypocellular but had no lymphomatous infiltration. This marrow sample contained 4% CAMPATH-1-positive cells and showed some oligoclonal rearrangements of immunoglobulin genes. However, by day 100, lymphoma cells were again detected in the blood and the spleen size had started to increase. A second course of 12 days' therapy with CAMPATH-1H was completed with similar therapeutic benefit to the first and no adverse effects. Since the main resevoir of disease in the patient appeared to be the spleen, splenectomy was carried out at the end of this second course of treatment. At that time no tumour cells could be detected in blood or marrow. The patient is now well 37 days after the splenectomy. The lymphocyte count is low but she has normal neutrophil, platelet, and red-cell counts.

Patient 2

A 67-year old man presented in April 1988 with splenic pain; there was 12 cm splenomegaly, and computed tomography scan of thorax and abdomen revealed retrocrural and para-aortic lymphadenopathy, the largest node measuring 3 cm in diameter (Figure 11C). A blood count revealed $36.6 \times 10^9$ lymphocytes/ml, the majority being lymphoplasmacytoid cells which expressed surface IgG-kappa and were characterised by large cytoplasmic periodic-acid-Schiff-positive vacuoles which could be intensely stained by anti-IgG. A marrow aspirate contained 72% lymphomatous cells (Figure 10C). DNA from blood mononuclear cells showed biallelic rearrangement of immunoglobulin $J_H$ genes but was germline with various T-cell receptor and oncogene probes. The lymphoma cells expressed the CAMPATH-1 antigen in amounts comparable with normal lymphocytes but were more resistant to complement-mediated lysis. Stage IVA grade I lymphoplasmacytoid non-Hodgkin lymphoma was diagnosed.

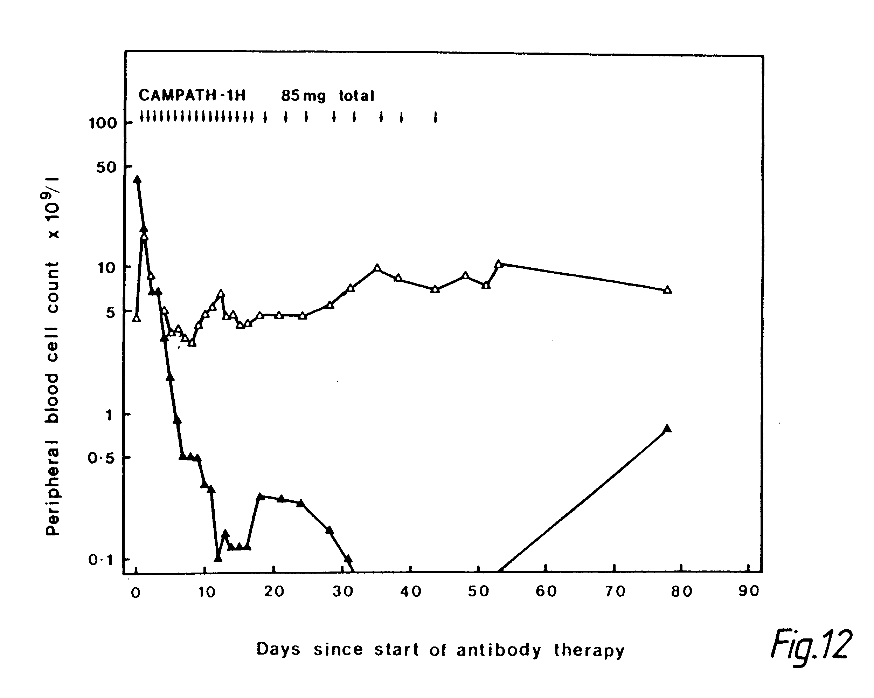

The patient was offered CAMPATH-1H as primary therapy and received a total of 85 mg over 43 days. This resulted in clearance of the lymphoma cells and normal lymphocytes from blood (Figure 12) and marrow (Figure 10D), resolution of splenomegaly, and improvement in the lymphadenopathy. A computed tomography scan taken 8 days after the end of antibody treatment was normal (Figure 11D). A bone marrow aspirate taken at the same time showed active haemopoiesis but no lymphoma cells, and no CAMPATH-1-positive cells could be detected by flow cytometry. DNA from the mononuclear fraction of this marrow showed only germline configuration when probed with the immunoglobulin $J_H$ probe. By day 78 morphologically normal blood lymphocytes began to reappear and none of the vacuolated cells could be seen. The patient remains well and off therapy.

Some toxic effects of CAMPATH-1H were observed. The first dose was stopped after 3 mg had been given because of hypotension, possibly caused by tumour lysis. This problem was subsequently avoided by giving smaller doses at a slower rate and when lymphoma cells had been cleared from the blood, the dose was increased to a maximun of 8 mg over 4 h without any effect on blood pressure. Nevertheless, all doses induced fever (up to 38.5°C), and malaise for up to 36 h, but these were not severe enough to stop antibody treatment which, after the first week, was given on an outpatient basis. Treatment was stopped after 43 days because of the development of an urticarial rash after two successive antibody infusions.

Half-life of CAMPATH 1-H

The concentration of CAMPATH-1H was measured in serum samples taken before and after antibody infusions and at other times throughout treatment. In theory, a dose of 4-6 mg corresponds to about 1 $\mu$g/ml in the plasma. In fact free antibody could not be detected until day 4-6, about 5-20% after 24 hours.

Lack of Antiglobulin Response

The allotype of CAMPATH-1H is Glm(1,2,17),Km(3). Patient 1 was Glm(1,3,17),Km(3) and patient 2 was Glm(3),Km(3), so both could theoretically have made an anti-allotype response as well as a response to the hypervariable regions. However, we failed to detect any antiglobulin to CAMPATH-1H either by the solid-phase enzyme-linked assay or by the more sensitive haemagglutination assay. In addition, the rate of clearance of CAMPATH-1H did not change and free antibody could be detected for up to 8 days after the last dose had been given. It is possible that the reactions experienced at the end of the course of treatment could have been provoked by contaminating non-human proteins in the antibody preparation.

Discussion

The remissions achieved in these two patients show that it is possible to clear large numbers of tumour cells with small amounts of an unmodified monoclonal antibody. The effects in the first patient were far superior to the results of the previous chemotherapy and radiotherapy. The selective lysis of lymphoma cells with recovery of normal haemopoiesis during the course of treatment was an important advantage, which allowed treatment to be given largely on an outpatient basis. It is believed the choice of antibody-specificity and isotype is important; indeed, it may be why these tests had more success than previous efforts with other monoclonal antibodies. (References 36-38.) The CAMPATH-1 antigen seems to be a good target because it is widely distributed and abundant, and does not suffer from antigenic modulation. (References 6,35.)

TABLE 3 - PATIENT CHARACTERISTICS BEFORE AND AFTER TREATMENT WITH CAMPATH-1H

|  | | Case 1 | | Case 2 | |
|---|---|---|---|---|---|
| Diagnosis | | Stage IVB grade I NHL in leukaemic phase | | Stage IVA grade I lympho-plasmacytoid NHL | |
|  | | before | after | before | after |
| Spleen size | ml | 4460 | 590 | 2600 | 440 |
| Lymphadenopathy | | nil | nil | retrocrural paraortic | nil |
| Bone marrow |  |  |  |  |  |
| lymphoma cell % | | 99 | 0 | 72 | 0 |
| Southern blot analysis |  |  |  |  |  |
| Ig $J_H$ fragment | | R/R | G/G | R/R | G/G |
| Peripheral blood |  |  |  |  |  |
| haemoglobin | g/dl | 8.7 | 10.6 | 11.2 | 12.0 |
| reticulocytes | x$10^9$/l | 31 | 135 | nd | nd |
| platelets | x$10^9$/l | 37 | 50 | 110 | 453 |
| lymphocytes | x$10^9$/l | 60 | 0 | 37 | 0 |
| neutrophils | x$10^9$/l | 0 | 2.0 | 4.6 | 7.3 |

| monocytes | x10$^9$/l | 0 | 0.04 | 1.5 | 0.5 |
|---|---|---|---|---|---|

**Lymphocyte phenotype**

| | | | | | |
|---|---|---|---|---|---|
| CD19 | % | 97 | 46 | 93 | <5 |
| CD3 | % | 0 | 32 | 8 | 80 |
| CAMPATH-1M | % | 96 | nd | 95 | nd |
| CAMPATH-1H | % | 98 | nd | 97 | nd |

**Serum immunoglobulins**

| | | | | | |
|---|---|---|---|---|---|
| IgM | g/l | <0.3 | 1.2 | <0.3 | 0.7 |
| IgA | g/l | <0.5 | <0.5 | <0.5 | 0.5 |
| IgG | g/l | 5.8 | 8.2 | 3.2 | 4.7 |
| Bence-Jones | | nil | nil | ++ | nil |

The post-therapy values refer to measurements made shortly after the end of antibody therapy, except for lymphocyte phenotyping and serum Ig, which were assessed 6 weeks later. Lymphocyte phenotypes were measured by immunofluorescence and APAAP techniques. Spleen and lymph node dimensions were measured by serial CT scanning (Fig11).

nd = not done

References

1. Neuberger, M.S., Williams, G.T., Mitchell, E.B., Jouhal, S.S., Flanagan, J.G. & Rabbits, T.H. Nature 314,268-270 (1985).
2. Liu, A.Y., Robinson, R.R., Hellstrom, K.E., Murray, E.D. Jr., Cheng, C.P. & Hellstrom, I. Proc.natl.Acad.Sci USA 84,3439-3443 (1987).
3. Shaw, D.R., Khazaeli, M.B., Sun, L.K., Ghraeyeb, J., Daddona, P.E., McKinney, S. & Lopuglio, A.F. J, Immunol. 138,4534-4538 (1987).
4. Bruggemann, M., Williams, G.T., Bindon, C., Clark, M.R., Walker, M.R., Jefferis, R., Waldmann, H. & Neuberger, M.S. J.Exp.Med. 166, 1351-1361 (1987).
5. Jones, P.T., Dear, P.H., Foote, J., Neuberger, M.S. & Winter, G. Nature 321,522-525 (1986).
6. Hale, G., Bright, S., Chumbley, G., Hoang, T., Metcalf, D., Munro, A.J. & Waldmann, H. Blood 62,873-882 (1983).
7. Hale, G., Hoang, T., Prospero, T., Watts, S.M. & Waldmann, H. Mol.Biol.Med. 1,305-319 (1983).
8. Hale, G., Cobbold, S.P., Waldmann, H., Easter, G., Matejtschuk, P. & Coombs, R.R.A. J. Immunol.Meth. 103,59-67 (1987).
9. Hale, G., Waldmann, H., Friend, P. & Calne, R. Transplantation 42,308-311 (1986).

10. Hale, G., Swirsky, D.M., Hayhoe, F.G.J. & Waldmann, H. Mol.Biol.Med. 1,321-334 (1983).

11. Kabat, E.A., Wu, T.T., Reid-Miller, M., Perry, H.M. & Gottesman, K.S. in Sequences of Proteins of Immunological Interest (US Dept. of Health and Human Services, 1987).

12. Chothia, C., Novotny, J., Bruccoleri, R. & Karplus, M. J. Mol.Biol. 186,651-663 (1985).

13. Saul, F.A., Amzel, M. & Poljak, R.J. J.Biol.Chem. 253,585-597 (1978).

14. EPP, O., Colman, P., Fehlhammer, H., Bode, W., Schiffer, M. & Huber, R. Eur.J.Biochem. 45,513-524 (1974).

15. Mulligan, R.C. & Berg, P. Proc.natl.Acad.Sci USA 78,2072-2076 (1981).

16. Southern, P.J. & Berg, P.J. Mol.Appl.Genetics 1,327-341 (1981).

17. Galfre, G. & Milstein, C. Meth.Enzymol. 73,1-46 (1981).

18. Chothia, C. & Lesk, A. J.Mol.Biol. 196,901-917 (1987).

19. Roberts, S., Cheetham, J.C. & Rees, A.R. Nature 328,731-734 (1987).

20. Jonker, M. & den Brok, J.H.A.M. Eur.J. Immunol. 17, 1547-1552 (1987).

21. Clark, M. & Waldmann, H. J.N.C.I. 79, 1393-1398 (1987).

22. Hieter, P.A., Max, E.E., Seidmann, J.G., Maizel, J.V.Jr & Leder, P. Cell 22,197-207 (1980).

23. Kaartinen, M., Griffiths, G.M., Hamlyn, P.H., Markham, A.F., Karjalainen, K., Pelkonen J.L.T., Makela, O. & Milstein, C.J. Immunol. 130,320-324 (1983).

24. Gubler, U. & Hoffmann, B.J. Gene 25,263-269 (1983).

25. Sanger, F., Nicklen, S.A. & Coulson, A.R. Proc.natl.Acad.Sci USA 74,5463-5467 (1977).

26. Takahashi, N., Ueda, N.S., Obata, M., Nikaido, T. & Honjo, T. Cell 29,671-679 (1982).

27. Flanagan, J.G. & Rabbits, T.H. Nature 300,709-713 (1982).

28. Huck, S., Fort, P., Crawford, D.H., Lefranc, M.-P. & Lefranc, G. Nucl.Acid Res. 14,1779-1789 (1986).

29. Bruggemann, M., Free, J., Diamond, A., Howard, J., Cobbold, S. & Waldmann, H. Proc.natl.Acad.Sci. USA 83,6075-6079 (1986).

30. Potter, H., Weir, L. & Leder, P. Proc.natl.Acad.Sci. USA 81,7161-7163 (1984).

31. Marquardt, M., Deisenhofer, J.,Huber, R. & Palm, W. J.Mol.Biol. 141,368-391 (1980).

32. Hale, G., Clark, M. & Waldmann, H. J. Immunol. 134,3056-3061 (1985).

33. Carter, P., Bedouelle, H. & Winter, G. Nucleic Acids Res. 13,4431-4443 (1985).

34. Heiter, P., Maizel, J & Leder, P. J. Biol. Chem. 257, 1516-1522 (1982).

35. Hale, G., Swirsky, D., Waldman, H., & Chan, L.C., Br. J. Haematol. 60, 41-48, (1985).

36. Ritz. J., Schlossman, S.F., Blood, 59, 1-11 (1982).

37. Levy. R., Miller R.A., Annu. Rev. Med., 34, 107-16 (1983).

38. Stevenson, G.T., Glennie, M.J., Cancer Surv. 4, 213-44, (1985).

## Claims

**Claims for the following Contracting States : DE, GB, FR, IT, NL, SE, LI, CH, BE, AT, LU**

1. An antibody with affinity for the antigen Campath-1 having at least one complementarity determining region which is foreign with respect to the constant region of the antibody, said at least one foreign complementarity determining region being selected from complementarity determining regions which are substantially as identified in Figure 2, that is amino acid residues 31 to 35, 50 to 65 and 95 to 102 of the heavy chain as shown in Figure 2a, and amino acid residues 24 to 34, 50 to 56 and 89 to 97 of the light chain as shown in Figure 2b, the antibody being capable of binding effectively to the antigen Campath-1.

2. An antibody according to Claim 1 having a heavy chain with at least one complementarity determining region selected from complementarity determining regions which are substantially as identified in Figure 2a and a light chain with at least one complementarity determining region selected from complementarity determining regions which are substantially as identified in Figure 2b.

3. An antibody according to Claim 1 having a heavy chain with three complementarity determining regions which are substantially as identified in Figure 2a or a light chain with three complementarity determining regions which are substantially as identified in Figure 2b.

4. An antibody with affinity for the antigen Campath-1 having heavy and light chain complementarity determining regions which are foreign with respect to the constant region of the antibody, said complementarity determining regions being substantially as identified in Figure 2, that is residues 31 to 35, 50 to 65 and 95 to 102 of the heavy chain as shown in Figure 2a, and residues 24 to 34, 50 to 56

16

and 89 to 97 of the light chain as shown in Figure 2b, the antibody being capable of binding effectively to the antigen Campath-1.

5. An antibody according to Claim 4 which has heavy and light chain variable domains as identified in the lower lines of sequence information in Figure 2, that is residues 1 to 113 of the heavy chain and residues 1 to 108 of the light chain.

6. An antibody according to any of Claims 1 to 4, wherein the complementarity determining regions are combined with heavy and light chain variable domain framework regions which are of human origin.

7. An antibody with affinity for the antigen Campath-1 having heavy and light chain complementarity determining regions which are foreign with respect to the constant region of the antibody, said complementarity determining regions being as identified in Figure 2, that is residues 31 to 35, 50 to 65 and 95 to 102 of the heavy chain as shown in Figure 2a, and residues 24 to 34, 50 to 56 and 89 to 97 of the light chain as shown in Figure 2b, and said complementarity determining regions being combined with heavy and light chain variable domain framework regions which are of human origin, the antibody being capable of binding effectively to the antigen Campath-1.

8. An antibody according to Claim 7 which has heavy and light chain variable domains as identified in the upper lines of sequence information in Figure 2, that is residues 1 to 113 of the heavy chain and residues 1 to 108 of the light chain.

9. An antibody as defined in Claim 8 but which has phenylalanine at residue 27 in the heavy chain in place of serine.

10. An antibody as defined in Claim 8 but which has phenylalanine at residue 27 and threonine at residue 30 in the heavy chain in place of serine.

11. An antibody according to any of the preceding claims, wherein the heavy chain constant domains and the light chain constant domain are selected from antibodies of different species type.

12. An antibody according to Claim 11, wherein said different species types include rat and human.

13. An antibody according to any of Claims 1 to 8, wherein the heavy and light chain constant domains are of human origin.

14. An antibody according to Claim 13, wherein the constant region is of an IgG class.

15. An antibody according to Claim 14, wherein the constant region is of the IgG1 class.

16. An antibody with affinity for the antigen Campath-1 having heavy and light chain variable domains as identified in the upper lines of sequence information in Figure 2, that is residues 1 to 113 of the heavy chain and residues 1 to 108 of the light chain, but with the serine at residue 27 in the heavy chain replaced by phenylalanine, and having heavy and light chain constant domains which are of human origin.

17. An antibody as defined in Claim 16 but which has threonine at residue 30 in the heavy chain in place of serine.

18. An antibody according to Claim 16 or 17, wherein the constant region is of an IgG class.

19. An antibody according to Claim 18, wherein the constant region is of the IgG1 class.

20. A fragment of an antibody according to any of the preceding claims, which fragment retains the binding capability of the antibody.

21. A composition for administration to patients comprising an antibody according to any of Claims 1 to 19 or a fragment according to Claim 20 together with a physiologically acceptable diluent or carrier.

17

22. A composition according to Claim 21, wherein said antibody or fragment is of a purity such that it is at least 95% by weight free of other biological materials.

23. A composition according to Claim 21 or 22 which is adapted for intravenous administration.

24. A composition according to any of Claims 21 to 23 which additionally comprises another therapeutic antibody.

25. A composition according to any of Claims 21 to 24 which comprises a unit dosage of 1 to 20 mg of the antibody with affinity for the antigen Campath-1 or of the fragment thereof.

26. A composition according to Claim 25, in which the unit dosage is 5 mg.

27. An antibody according to any of Claims 1 to 19 or a fragment according to Claim 20 for use in therapy.

28. The use of an antibody according to any of Claims 1 to 19 or of a fragment according to Claim 20 in the manufacture of a medicament for use in a therapy which requires reduction of the lymphocyte population.

29. The use of an antibody according to any of Claims 1 to 19 or of a fragment according to Claim 20 in the manufacture of a medicament for use in the treatment of cancer.

30. The use of an antibody according to any of Claims 1 to 19 or of a fragment according to Claim 20 in the manufacture of a medicament for use in immunosuppression.

31. The use according to Claim 29 or 30, in which the medicament is for use in the treatment of lymphoid malignancies.

32. The use according to Claim 31, in which the lymphoid malignancy is a lymphoma.

33. The use according to Claim 32, in which the medicament is in a unit dosage form containing 1 to 20 mg of the antibody or fragment thereof.

34. The use according to Claim 33, in which the medicament is in a unit dosage form containing 5 mg of the antibody or fragment thereof.

35. The use according to Claim 30, in which the medicament is for use in the treatment of autoimmune disease.

36. The use according to Claim 30, in which the medicament is for use in the treatment of a recipient of a transplant.

37. A cell line which expresses an antibody with affinity for the antigen Campath-1 having at least one complementarity determining region which is foreign with respect to the constant region of the antibody, said at least one foreign complementarity determining region being selected from complementarity determining regions which are substantially as identified in Figure 2, that is amino acid residues 31 to 35, 50 to 65 and 95 to 102 of the heavy chain as shown in Figure 2a, and amino acid residues 24 to 34, 50 to 56 and 89 to 97 of the light chain as shown in Figure 2b, the antibody being capable of binding effectively to the antigen Campath-1.

38. A cell line according to Claim 37, wherein the antibody is as defined in any of Claims 2 to 20.

39. A cell line according to Claim 37 or 38, wherein the cell line comprises a DNA sequence or sequences shown in Figure 2 as corresponding to one or more of said complementarity determining regions.

**Claims for the following Contracting States : ES, GR**

1. A process for the preparation of an antibody with affinity for the antigen Campath-1 having at least one complementarity determining region which is foreign with respect to the constant region of the antibody, said at least one foreign complementarity determining region being selected from complementarity determining regions which are substantially as identified in Figure 2, that is amino acid residues 31 to 35, 50 to 65 and 95 to 102 of the heavy chain as shown in Figure 2a, and amino acid residues 24 to 34, 50 to 56 and 89 to 97 of the light chain as shown in Figure 2b, the antibody being capable of binding effectively to the antigen Campath-1, which process comprises culturing cells capable of expressing the antibody in order to effect expression thereof.

2. A process according to Claim 1, wherein the antibody has a heavy chain with at least one complementarity determining region selected from complementarity determining regions which are substantially as identified in Figure 2a and a light chain with at least one complementarity determining region selected from complementarity determining regions which are substantially as identified in Figure 2b.

3. A process according to Claim 1, wherein the antibody has a heavy chain with three complementarity determining regions which are substantially as identified in Figure 2a or a light chain with three complementarity determining regions which are substantially as identified in Figure 2b.

4. A process for the preparation of an antibody with affinity for the antigen Campath-1 having heavy and light chain complementarity determining regions which are foreign with respect to the constant region of the antibody, said complementarity determining regions being substantially as identified in Figure 2, that is residues 31 to 35, 50 to 65 and 95 to 102 of the heavy chain as shown in Figure 2a, and residues 24 to 34, 50 to 56 and 89 to 97 of the light chain as shown in Figure 2b, the antibody being capable of binding effectively to the antigen Campath-1, which process comprises culturing cells capable of expressing the antibody in order to effect expression thereof.

5. A process according to Claim 4, wherein the antibody has heavy and light chain variable domains as identified in the lower lines of sequence information in Figure 2, that is residues 1 to 113 of the heavy chain and residues 1 to 108 of the light chain.

6. A process according to any of Claims 1 to 4, wherein the complementarity determining regions of the antibody are combined with heavy and light chain variable domain framework regions which are of human origin.

7. A process for the preparation of an antibody with affinity for the antigen Campath-1 having heavy and light chain complementarity determining regions which are foreign with respect to the constant region of the antibody, said complementarity determining regions being as identified in Figure 2, that is residues 31 to 35, 50 to 65 and 95 to 102 of the heavy chain as shown in Figure 2a, and residues 24 to 34, 50 to 56 and 89 to 97 of the light chain as shown in Figure 2b, and said complementarity determining regions being combined with heavy and light chain variable domain framework regions which are of human origin, the antibody being capable of binding effectively to the antigen Campath-1, which process comprises culturing cells capable of expressing the antibody in order to effect expression thereof.

8. A process according to Claim 7, wherein the antibody has heavy and light chain variable domains as identified in the upper lines of sequence information in Figure 2, that is residues 1 to 113 of the heavy chain and residues 1 to 108 of the light chain.

9. A process according to Claim 7, wherein the antibody is as defined in Claim 8 but has phenylalanine at residue 27 in the heavy chain in place of serine.

10. A process according to Claim 7, wherein the antibody is as defined in Claim 8 but has phenylalanine at residue 27 and threonine at residue 30 in the heavy chain in place of serine.

11. A process according to any of the preceding claims, wherein the heavy chain constant domains and the light chain constant domain of the antibody are selected from antibodies of different species type.

**12.** A process according to Claim 11, wherein said different species types include rat and human.

**13.** A process according to any of Claims 1 to 8, wherein the heavy and light chain constant domains of the antibody are of human origin.

**14.** A process according to Claim 13, wherein the constant region of the antibody is of an IgG class.

**15.** A process according to Claim 14, wherein the constant region of the antibody is of the IgG1 class.

**16.** A process for the preparation of an antibody with affinity for the antigen Campath-1 having heavy and light chain variable domains as identified in the upper lines of sequence information in Figure 2, that is residues 1 to 113 of the heavy chain and residues 1 to 108 of the light chain, but with the serine at residue 27 in the heavy chain replaced by phenylalanine, and having heavy and light chain constant domains which are of human origin, which process comprises culturing cells capable of expressing the antibody in order to effect expression thereof.

**17.** A process according to Claim 16, wherein the antibody is as defined in Claim 16 but has threonine at residue 30 in the heavy chain in place of serine.

**18.** A process according to Claim 16 or 17, wherein the constant region of the antibody is of an IgG class.

**19.** A process according to Claim 18, wherein the constant region of the antibody is of the IgG1 class.

**20.** A process according to any of the preceding claims, wherein the antibody produced is treated to produce a fragment which retains the binding capability of the antibody.

**21.** A process for the preparation of a composition for administration to patients containing an antibody as defined in any of Claims 1 to 19 or a fragment as defined in Claim 20 which comprises bringing the antibody or fragment together with a physiologically acceptable diluent or carrier.

**22.** A process according to Claim 21, wherein said antibody or fragment is of a purity such that it is at least 95% by weight free of other biological materials.

**23.** A process according to Claim 21 or 22, wherein the composition is adapted for intravenous administration.

**24.** A process according to any of Claims 21 to 23, wherein the composition additionally comprises another therapeutic antibody.

**25.** A process according to any of Claims 21 to 24, wherein the composition comprises a unit dosage of 1 to 20 mg of the antibody with affinity for the antigen Campath-1 or of the fragment thereof.

**26.** A process according to Claim 25, in which the unit dosage is 5 mg.

**27.** A cell line which expresses an antibody with affinity for the antigen Campath-1 having at least one complementarity determining region which is foreign with respect to the constant region of the antibody, said at least one foreign complementarity determining region being selected from complementarity determining regions which are substantially as identified in Figure 2, that is amino acid residues 31 to 35, 50 to 65 and 95 to 102 of the heavy chain as shown in Figure 2a, and amino acid residues 24 to 34, 50 to 56 and 89 to 97 of the light chain as shown in Figure 2b, the antibody being capable of binding effectively to the antigen Campath-1.

**28.** A cell line according to Claim 27, wherein the antibody is as defined in any of Claims 2 to 20.

**29.** A cell line according to Claim 27 or 28, wherein the cell line comprises a DNA sequence or sequences shown in Figure 2 as corresponding to one or more of said complementarity determining regions.

**Patentansprüche**

**Patentansprüche für folgende Vertragsstaaten : DE, GB, FR, LT, NL, SE, LI, CH, BE, AT, LU**

1. Antikörper mit Affinität für das Campath-1-Antigen mit mindestens einer komplementären determinierenden Region, die bezüglich der konstanten Region des Antikörpers fremd ist, wobei die mindestens eine fremde komplementäre determinierende Region aus den komplementären determinierenden Regionen, die im Wesentlichen wie in Figur 2 dargestellt sind, ausgewählt ist, das heißt, die Aminosäurereste 31 bis 35, 50 bis 65 und 95 bis 102 der schweren Kette, wie in Figur 2a gezeigt, und die Aminosäurereste 24 bis 34, 50 bis 56 und 89 bis 97 der leichten Kette wie in Figur 2b gezeigt, wobei der Antikörper effektiv an das Campath-1-Antigen binden kann.

2. Antikörper nach Anspruch 1, dadurch **gekennzeichnet**, daß er eine schwere Kette mit mindestens einer komplementären determinierenden Region, ausgewählt aus den komplementären determinierenden Regionen, die, im wesentlichen wie in Figur 2a dargestellt, sind, und eine leichte Kette mit mindestens einer komplementären determinierenden Region, ausgewählt aus den komplementären determinierenden Regionen, die, im wesentlichen wie in Figur 2b dargestellt, sind, besitzt.

3. Antikörper nach Anspruch 1, dadurch **gekennzeichnet**, daß er eine schwere Kette mit drei komplementären determinierenden Regionen, die, im wesentlichen wie in Figur 2a dargestellt, sind, oder eine leichte Kette mit drei komplementären determinierenden Regionen, die im wesentlichen wie in Figur 2b dargestellt sind, besitzt.

4. Antikörper mit Affinität für das Campath-1-Antigen mit komplementären determinierenden Regionen der schweren und der leichten Kette, die bezüglich der konstanten Region des Antikörpers fremd sind, wobei die komplementären determinierenden Regionen im wesentlichen wie in Figur 2 dargestellt sind, das heißt, die Reste 31 bis 35, 50 bis 65 und 95 bis 102 der schweren Kette, wie in Figur 2a dargestellt, und die Reste 24 bis 34, 50 bis 56 und 89 bis 97 der leichten Kette, wie in Figur 2b dargestellt, wobei der Antikörper effektiv an das Campath-1-Antigen binden kann.

5. Antikörper nach Anspruch 4, dadurch **gekennzeichnet**, daß er variable Domänen der schweren und der leichten Kette, wie in den unteren Zeilen der Sequenzinformation in Figur 1 dargestellt ist, besitzt, das heißt, die Reste 1 bis 113 der schweren Kette und die Reste 1 bis 108 der leichten Kette.

6. Antikörper nach einem der Ansprüche 1 bis 4, dadurch **gekennzeichnet**, daß die komplementären determinierenden Regionen mit Gerüstregionen der variablen Domäne der schweren und leichten Kette, die menschlichen Ursprungs sind, kombiniert sind.

7. Antikörper mit Affinität für das Campath-1-Antigen mit komplementären determinierenden Regionen der schweren und der leichten Kette, die bezüglich der konstanten Region des Antikörpers fremd sind, wobei die komplementären determinierenden Regionen wie in Figur 2 dargestellt sind, das heißt, die Reste 31 bis 35, 50 bis 65 und 95 bis 102 der schweren Kette, wie in Figur 2a gezeigt, und die Reste 24 bis 34, 50 bis 56 und 89 bis 97 der leichten Kette, wie in Figur 2b gezeigt, und wobei die komplementären determinierenden Regionen mit Gerüstregionen der variablen Domäne der schweren und der leichten Kette, die menschlichen Ursprungs sind, kombiniert sind, wobei der Antikörper effektiv an das Campath-1-Antigen binden kann.

8. Antikörper nach Anspruch 1, dadurch **gekennzeichnet**, daß er variable Domänen der schweren und der leichten Kette, wie in der oberen Zeile der Sequenzinformation in Figur 2 dargestellt, besitzt, das heißt, die Reste 1 bis 113 der schweren Kette und die Reste 1 bis 108 der leichten Kette.

9. Antikörper nach Anspruch 8, jedoch mit Phenylalanin bei Rest 27 in der schweren Kette anstelle von Serin.

10. Antikörper nach Anspruch 8, jedoch mit Phenylalanin bei Rest 27 und Threonin bei Rest 30 in der schweren Kette anstelle von Serin.

11. Antikörper nach einem der vorausgehenden Ansprüche, dadurch **gekennzeichnet**, daß die konstanten Domänen der schweren Kette und die konstante Domäne der leichten Kette aus Antikörpern von

EP 0 328 404 B1

unterschiedlichen Speziesarten ausgewählt sind.

12. Antikörper nach Anspruch 11, dadurch **gekennzeichnet**, daß die unterschiedlichen Speziesarten Ratte und Mensch umfassen.

13. Antikörper nach einem der Ansprüche 1 bis 8, dadurch **gekennzeichnet**, daß die konstanten Domänen der schweren und der leichten Kette menschlichen Ursprungs sind.

14. Antikörper nach Anspruch 13, dadurch **gekennzeichnet**, daß die konstante Region einer IgG-Klasse angehört.

15. Antikörper nach Anspruch 14, dadurch **gekennzeichnet**, daß die konstante Region der IgG1-Klasse angehört.

16. Antikörper mit Affinität für das Campath-1-Antigen mit variablen Domänen der schweren und der leichten Kette, wie in den oberen Zeilen der Sequenzinformation in Figur 2 dargestellt, das heißt, die Reste 1 bis 113 der schweren Kette und die Reste 1 bis 108 der leichten Kette, jedoch mit einem Austausch von Serin bei Rest 27 in der schweren Kette durch Phenylalanin und mit konstanten Domänen der schweren und der leichten Kette, die menschlichen Ursprungs sind.

17. Antikörper nach Anspruch 16, jedoch mit Threonin bei Rest 30 in der schweren Kette anstelle von Serin.

18. Antikörper nach Anspruch 16 oder 17, dadurch **gekennzeichnet**, daß die konstante Region einer IgG-Klasse angehört.

19. Antikörper nach Anspruch 18, dadurch **gekennzeichnet**, daß die konstante Region der IgG1-Klasse angehört.

20. Fragment eines Antikörpers nach einem der vorausgehenden Ansprüche, dadurch **gekennzeichnet**, daß das Fragment die Bindungsfähigkeit des Antikörpers beibehält.

21. Präparat zur Verabreichung an Patienten, umfassend einen Antikörper nach einem der Ansprüche 1 bis 19 oder ein Fragment nach Anspruch 20 zusammen mit einem physiologisch verträglichen Verdünnungsmittel oder Träger.

22. Präparat nach Anspruch 21, dadurch **gekennzeichnet**, daß der Antikörper oder das Fragment so rein ist, daß mindestens 95 Gew.-% frei von anderen biologischen Materialien sind.

23. Präparat nach Anspruch 21 oder 22, dadurch **gekennzeichnet**, daß es der intravenösen Verabreichung angepaßt ist.

24. Präparat nach einem der Ansprüche 21 bis 23, dadurch **gekennzeichnet**, daß es weiterhin einen anderen therapeutischen Antikörper enthält.

25. Präparat nach einem der Ansprüche 21 bis 24, dadurch **gekennzeichnet**, daß es eine Dosiseinheit von 1 bis 20 mg des Antikörpers mit Affinität für das Campath-1-Antigen oder dem Fragment davon enthält.

26. Präparat nach Anspruch 25, dadurch **gekennzeichnet**, daß die Dosiseinheit 5 mg beträgt.

27. Antikörper nach einem der Ansprüche 1 bis 19, oder ein Fragment nach Anspruch 20 zur therapeutischen Verwendung.

28. Verwendung eines Antikörpers nach einem der Ansprüche 1 bis 19 oder eines Fragments nach Anspruch 20 zur Herstellung eines Medikaments zur Verwendung in einer Therapie, die die Verringerung der Lymphozytenpopulation notwending macht.

22

EP 0 328 404 B1

**29.** Verwendung eines Antikörpers nach einem der Ansprüche 1 bis 19 oder eines Fragments nach Anspruch 20 zur Herstellung eines Medikaments zur Verwendung bei der Behandlung von Krebs.

**30.** Verwendung eines Antikörpers nach einem der Ansprüche 1 bis 19 oder eines Fragments nach Anspruch 20 zur Herstellung eines Medikaments zur Verwendung bei der Immunsuppression.

**31.** Verwendung nach Anspruch 29 oder 30, dadurch **gekennzeichnet**, daß das Medikament bei der Behandlung von lymphoiden Malignitäten verwendet wird.

**32.** Verwendung nach Anspruch 31, dadurch **gekennzeichnet**, daß die lymphoide Malignität ein Lymphom ist.

**33.** Verwendung nach Anspruch 32, dadurch **gekennzeichnet**, daß das Medikament in einer Einheitsdosisform, die 1 bis 20 mg des Antikörpers oder Fragments davon enthält, vorliegt.

**34.** Verwendung nach Anspruch 33, daduch **gekennzeichnet**, daß das Medikament in einer Dosiseinheitsform, die 5 mg des Antikörpers oder Fragments davon enthält, vorliegt.

**35.** Verwendung nach Anspruch 30, dadurch **gekennzeichnet**, daß das Medikament bei der Behandlung einer Autoimmunerkrankung verwendet wird.

**36.** Verwendung nach Anspruch 30, dadurch **gekennzeichnet**, daß das Medikament bei der Behandlung eines Transplantatempfängers verwendet wird.

**37.** Zellinie, die einen Antikörper mit Affinität für das Campath-1-Antigen mit mindestens einer komplementären determinierenden Region, die bezüglich der konstanten Region des Antikörpers fremd ist, exprimiert, wobei mindestens eine fremde komplementäre determinierende Region aus den komplementären determinierenden Regionen, die im wesentlichen wie in Figur 2 dargestellt sind, ausgewählt ist, das heißt, die Aminosäurereste 31 bis 35, 50 bis 65 und 95 bis 102 der schweren Kette, wie in Figur 2a gezeigt, und die Aminosäurereste 24 bis 34, 50 bis 56 und 89 bis 97 der leichten Kette, wie in Figur 2b gezeigt, wobei der Antikörper effektiv an das Campath-1-Antigen binden kann.

**38.** Zellinie nach Anspruch 37, dadurch **gekennzeichnet**, daß der Antikörper wie in einem der Ansprüche 2 bis 20 definiert ist.

**39.** Zellinie nach Anspruch 37 oder 38, dadurch **gekennzeichnet**, daß die Zellinie eine DNA-Sequenz oder DNA-Sequenzen, die in Figur 2 gezeigt sind, entsprechend einer oder mehrerer komplementärer determinierender Regionen umfaßt.

**Patentansprüche für folgende Vertragsstaaten : ES, GR**

**1.** Verfahren zur Herstellung eines Antikörpers mit Affinität für das Campath-1-Antigen mit mindestens einer komplementären determinierenden Region, die bezüglich der konstanten Region des Antikörpers fremd ist, wobei die mindestens eine fremde komplementäre determinierende Region aus den komplementären determinierenden Regionen, die im wesentlichen wie in Figur 2 dargestellt sind, ausgewählt ist, das heißt, die Aminosäurereste 31 bis 35, 50 bis 65 und 95 bis 102 der schweren Kette, wie in Figur 2a gezeigt, und die Aminosäurereste 24 bis 34, 50 bis 56 und 89 bis 97 der leichten Kette, wie in Figur 2b gezeigt, wobei der Antikörper effektiv an das Campath-1-Antigen binden kann, dadurch **gekennzeichnet**, daß man Zellen mit der Fähigkeit, den Antikörper zu exprimieren, züchtet, um die Expression davon zu bewirken.

**2.** Verfahren nach Anspruch 1, dadurch **gekennzeichnet**, daß der Antikörper eine schwere Kette mit mindestens einer komplementären determinierenden Region, ausgewählt aus den komplementären determinierenden Regionen, die im wesentlichen wie in Figur 2a dargestellt sind, und eine leichte Kette mit mindestens einer komplementären determinierenden Region, ausgewählt aus den komplementären determinierenden Regionen, die im wesentlichen wie in Figur 2b dargestellt sind, besitzt.

23

3. Verfahren nach Anspruch 1, dadurch **gekennzeichnet**, daß der Antikörper eine schwere Kette mit drei komplementären determinierenden Regionen, die im wesentlichen wie in Figur 2a dargestellt sind, oder eine leichte Kette mit drei komplementären determinierenden Regionen, die im wesentlichen wie in Figur 2b dargestellt sind, besitzt.

4. Verfahren zur Herstellung eines Antikörpers mit Affinität für das Campath-1-Antigen mit komplementären determinierenden Regionen der schweren und der leichten Kette, die bezüglich der konstanten Region des Antikörpers fremd sind, wobei die komplementären determinierenden Regionen im wesentlichen wie in Figur 2 dargestellt sind, das heißt, die Reste 31 bis 35, 50 bis 65 und 95 bis 102 der schweren Kette, wie in Figur 2a gezeigt, und die Reste 24 bis 34, 50 bis 56 und 89 bis 97 der leichten Kette, wie in Figur 2b gezeigt, wobei der Antikörper effektiv an das Campath-1-Antigen binden kann, dadurch **gekennzeichnet**, daß man Zellen mit der Fähigkeit zur Expression des Antikörpers züchtet, um die Expression davon zu bewirken.

5. Verfahren nach Anspruch 4, dadurch **gekennzeichnet**, daß der Antikörper variable Domänen der schweren und der leichten Kette, wie in den unteren Zeilen der Sequenzinformation in Figur 2 gezeigt, besitzt, das heißt, die Reste 1 bis 113 der schweren Kette und die Reste 1 bis 108 der leichten Kette.

6. Verfahren nach einem der Ansprüche 1 bis 4, dadurch **gekennzeichnet**, daß die komplementären determinierenden Regionen des Antikörpers mit Gerüstregionen der variablen Domäne der schweren und der leichten Kette, die menschlichen Ursprungs sind, kombiniert sind.

7. Verfahren zur Herstellung eines Antikörpers mit Affinität für das Campath-1-Antigen mit komplementären determinierenden Regionen der schweren und der leichten Kette, die bezüglich der konstanten Region des Antikörpers fremd sind, wobei die komplementären determinierenden Regionen wie in Figur 2 dargestellt sind, das heißt die Reste 31 bis 35, 50 bis 56 und 95 bis 102 der schweren Kette, wie in Figur 2a gezeigt, und die Reste 24 bis 34, 50 bis 56 und 89 bis 97 der leichten Kette, wie in Figur 2b gezeigt, und wobei die komplementären determinierenden Regionen mit Gerüstregionen der variablen Domäne der schweren und der leichten Kette, die menschlichen Ursprungs sind, kombiniert sind, wobei der Antikörper effektiv an das Campath-1-Antigen binden kann, dadurch **gekennzeichnet**, daß man Zellen mit der Fähigkeit zur Expression des Antikörpers züchtet, um die Expression davon zu bewirken.

8. Verfahren nach Anspruch 7, dadurch **gekennzeichnet**, daß der Antikörper variable Domänen der schweren und der leichten Kette, wie in den oberen Zeilen der Sequenzinformation in Figur 2 gezeigt, besitzt, das heißt, die Reste 1 bis 113 der schweren Kette und die Reste 1 bis 108 der leichten Kette.

9. Verfahren nach Anspruch 7, dadurch **gekennzeichnet**, daß der Antikörper wie in Anspruch 8 definiert ist, aber Phenylalanin bei Rest 27 in der schweren Kette anstelle von Serin besitzt.

10. Verfahren nach Anspruch 7, dadurch **gekennzeichnet**, daß der Antikörper wie in Anspruch 8 definiert ist, aber Phenylalanin bei Rest 27 und Threonin bei Rest 30 in der schweren Kette anstelle von Serin besitzt.

11. Verfahren nach einem der vorausgehenden Ansprüche, dadurch **gekennzeichnet**, daß die konstanten Domänen der schweren Kette und die konstante Domäne der leichten Kette des Antikörpers aus Antikörpern unterschiedlicher Speziesarten ausgewählt sind.

12. Verfahren nach Anspruch 11, dadurch **gekennzeichnet**, daß die unterschiedlichen Speziesarten Ratte und Mensch umfassen.

13. Verfahren nach einem der Ansprüche 1 bis 8, dadurch **gekennzeichnet**, daß die konstanten Domänen der schweren und der leichten Kette des Antikörpers menschlichen Ursprungs sind.

14. Verfahren nach Anspruch 13, dadurch **gekennzeichnet**, daß die konstante Region des Antikörpers einer IgG-Klasse angehört.

**15.** Verfahren nach Anspruch 14, dadurch **gekennzeichnet**, daß die konstante Region des Antikörpers der IgG1-Klasse angehört.

**16.** Verfahren zur Herstellung eines Antikörpers mit Affinität für das Campath-1-Antigen mit variablen Domänen der schweren und der leichten Kette, wie in den oberen Zeilen der Sequenzinformation in Figur 2 dargestellt, das heißt, die Reste 1 bis 113 der schweren Kette und die Reste 1 bis 108 der leichten Kette, aber mit einem Austausch von Serin bei Rest 27 in der schweren Kette gegen Phenylalanin und mit konstanten Domänen der schweren und der leichten Kette, die menschlichen Ursprungs sind, dadurch **gekennzeichnet**, daß man Zellen mit der Fähigkeit zur Expression des Antikörpers züchtet, um die Expression davon zu bewirken.

**17.** Verfahren nach Anspruch 16, dadurch **gekennzeichnet**, daß der Antikörper wie in Anspruch 16 definiert ist, aber Threonin bei Rest 30 in der schweren Kette anstelle von Serin besitzt.

**18.** Verfahren nach Anspruch 16 oder 17, dadurch **gekennzeichnet**, daß die konstante Region des Antikörpers einer IgG-Klasse angehört.

**19.** Verfahren nach Anspruch 18, dadurch **gekennzeichnet**, daß die konstante Region des Antikörpers der IgG1-Klasse angehört.

**20.** Verfahren nach einem der vorausgehenden Ansprüche, dadurch **gekennzeichnet**, daß der gebildete Antikörper behandelt wird, um ein Fragment, das die Bindungsfähigkeit des Antikörpers beibehält, zu erzeugen.

**21.** Verfahren zur Herstellung eines Präparats zur Verabreichung an Patienten, welches einen Antikörper nach einem der Ansprüche 1 bis 19 oder ein Fragment nach Anspruch 20 umfaßt, dadurch **gekennzeichnet**, daß man den Antikörper oder das Fragment mit einem physiologisch annehmbaren Verdünnungsmittel oder Träger vermischt.

**22.** Verfahren nach Anspruch 21, dadurch **gekennzeichnet**, daß der Antikörper oder das Fragment so rein ist, daß mindestens 95 Gew.-% frei von anderen biologischen Materialien sind.

**23.** Verfahren nach Anspruch 21 oder 22, dadurch **gekennzeichnet**, daß das Präparat der intravenösen Verabreichung angepaßt ist.

**24.** Verfahren nach einem der Ansprüche 21 bis 23, dadurch **gekennzeichnet**, daß das Präparat zusätzlich einen weiteren therapeutischen Antikörper enthält.

**25.** Verfahren nach einem der Ansprüche 21 bis 24, dadurch **gekennzeichnet**, daß das Präparat eine Dosiseinheit von 1 bis 20 mg des Antikörpers mit Affinität für das Campath-1-Antigen oder des Fragments davon, umfaßt.

**26.** Verfahren nach Anspruch 25, dadurch **gekennzeichnet**, daß die Dosiseinheit 5 mg beträgt.

**27.** Zellinie, die einen Antikörper mit Affinität für das Campath-1-Antigen mit mindestens einer komplementären determinierenden Region, die bezüglich der konstanten Region des Antikörpers fremd ist, exprimiert, wobei mindestens eine fremde komplementäre determinierende Region aus den komplementären determinierenden Regionen, die im wesentlichen wie in Figur 2 dargestellt sind, ausgewählt ist, das heißt, die Aminosäurereste 31 bis 35, 50 bis 65 und 95 bis 102 der schweren Kette, wie in Figur 2a gezeigt, und die Aminosäurereste 24 bis 34, 50 bis 56 und 89 bis 97 der leichten Kette, wie in Figur 2b gezeigt, wobei der Antikörper effektiv an das Campath-1-Antigen binden kann.

**28.** Zellinie nach Anspruch 27, daduch **gekennzeichnet**, daß der Antikörper wie in einem der Ansprüche 2 bis 20 definiert ist.

**29.** Zellinie nach Anspruch 27 oder 28, dadurch **gekennzeichnet**, daß die Zellinie eine DNA-Sequenz oder DNA-Sequenzen, wie in Figur 2 gezeigt, entsprechend einer oder mehreren der komplementären determinierenden Regionen umfaßt.

EP 0 328 404 B1

**Revendications**
**Revendications pour les Etats contractants suivants : DE , GB, FR, IT, NL, SE, LI, CH, BE, AT, LU**

**1.** Anticorps avec affinité pour l'antigène Campath-1 ayant au moins une région déterminante de complémentarité, qui est étrangère par rapport à la région constante de l'anticorps, cette(ces) région(s) déterminante(s) de complémentarité étrangère(s) étant choisie(s) parmi des régions déterminantes de complémentarité qui sont pratiquement comme identifié sur la figure 2, c'est-à-dire les résidus d'acides aminés 31 à 35, 50 à 65 et 95 à 102 de la chaîne lourde représentée sur la figure 2a, et les résidus d'acides aminés 24 à 34, 50 à 56 et 89 à 97 de la chaîne légère représentée sur la figure 2b, l'anticorps étant susceptible de se lier effectivement a l'antigène Campath-1.

**2.** Anticorps selon la revendication 1, ayant une chaîne lourde avec au moins une région déterminante de complémentarité choisie parmi des régions déterminantes de complémentarité qui sont pratiquement comme identifié sur la figure 2a et une chaîne légère avec au moins une région déterminante de complémentarité choisie parmi des régions déterminantes de complémentarité qui sont pratiquement comme identifié sur la figure 2b.

**3.** Anticorps selon la revendication 1, ayant une chaîne lourde avec trois régions déterminantes de complémentarité qui sont pratiquement comme identifié sur la figure 2a ou une chaîne légère avec trois régions déterminantes de complémentarité qui sont pratiquement comme identifié sur la figure 2b.

**4.** Anticorps avec une affinité pour l'antigène Campath-1 ayant des régions déterminantes de complémentarité de chaînes lourde et légère qui sont étrangères par rapport à la région constante de l'anticorps, ces régions déterminantes de complémentarité étant pratiquement comme identifié sur la figure 2, c'est-à-dire les résidus 31 à 35, 50 à 65 et 95 à 102 de la chaîne lourde représentée sur la figure 2a, et les résidus 24 à 34, 50 à 56 et 89 à 97 de la chaîne légère représentée sur la figure 2b, l'anticorps étant susceptible de se lier effectivement à l'antigène Campath-1.

**5.** Anticorps selon la revendication 4, qui a des domaines variables de chaînes lourde et légère comme identifié dans les lignes inférieures de l'information de séquence sur la figure 2, c'est-à-dire les résidus 1 à 113 de la chaîne lourde et les résidus 1 à 108 de la chaîne légère.

**6.** Anticorps selon l'une des revendications 1 à 4, dans lequel les régions déterminantes de complémentarité sont combinées avec des régions de cadre de domaines variables de chaînes lourde et légère qui sont d'origine humaine.

**7.** Anticorps avec une affinité pour l'antigène Campath-1 ayant des régions déterminantes de complémentarité de chaînes lourde et légère qui sont étrangères par rapport à la région constante de l'anticorps, ces régions déterminantes de complémentarité étant comme identifié sur la figure 2, c'està-dire les résidus 31 à 35, 50 à 65 et 95 à 102 de la chaîne lourde représentée sur la figure 2a et les résidus 24 à 34, 50 à 56 et 89 à 97 de la chaîne légère représentée sur la figure 2b, ces régions déterminantes de complémentarité étant combinées avec des régions de cadre de domaines variables de chaînes lourde et légère qui sont d'origine humaine, l'anticorps étant susceptible de se lier effectivement à l'antigène Campath-1.

**8.** Anticorps selon la revendication 7, qui a des domaines variables de chaînes lourde et légère comme identifié dans les lignes supérieures de l'information de séquence sur la figure 2, c'est-à-dire les résidus 1 à 113 de la chaîne lourde et les résidus 1 à 108 de la chaîne légère.

**9.** Anticorps selon la revendication 8, mais qui a phénylalanine au résidu 27 dans la chaîne lourde à la place de sérine.

**10.** Anticorps selon la revendication 8, mais qui a phénylalanine au résidu 27 et thréonine au résidu 30 dans la chaîne lourde à la place de sérine.

**11.** Anticorps selon l'une des revendications précédentes, dans lequel les domaines constants de la chaîne lourde et le domaine constant de la chaîne légère sont choisis à partir d'anticorps de différents types d'espèces.

26

**12.** Anticorps selon la revendication 11, dans lequel les types d'espèces différents comprennent le rat et l'homme.

**13.** Anticorps selon l'une des revendications 1 à 8, dans lequel les domaines constants des chaînes lourde et légère sont d'origine humaine.

**14.** Anticorps selon la revendication 13, dans lequel la région constante est d'une classe d'immunoglobulines.

**15.** Anticorps selon la revendication 14, dans lequel la région constante est de la classe IgG1.

**16.** Anticorps avec une affinité pour l'antigène Campath-1 ayant des domaines variables de chaînes lourde et légère comme identifié dans les lignes supérieures de l'information de séquence sur la figure 2, c'est-à-dire les résidus 1 à 113 de la chaîne lourde et les résidus 1 à 108 de la chaîne légère, mais avec la sérine au résidu 27 dans la chaîne lourde remplacée par la phénylalanine, et ayant des domaines constants de chaînes lourde et légère qui sont d'orignie humaine.

**17.** Anticorps selon la revendication 16, mais qui a thréonine au résidu 30 dans la chaîne lourde à la place de sérine.

**18.** Anticorps selon la revendication 16 ou la revendication 17, dans lequel la région constante est d'une classe IgG.

**19.** Anticorps selon la revendication 18, dans lequel la région constante est de la classe IgG1.

**20.** Fragment d'un anticorps selon l'une des revendications précédentes, lequel fragment contient la possibilité de liaison de l'anticorps.

**21.** Composition pour administration à des malades, contenant un anticorps selon l'une des revendications 1 à 19 ou un fragment selon la revendication 20 avec un diluant ou un porteur physiologiquement acceptable.

**22.** Composition selon la revendication 21, dans lequel l'anticorps ou le fragment a une pureté telle qu'il est au moins à 95% en poids dépourvu d'autres matériaux biologiques.

**23.** Composition selon la revendication 21 ou la revendication 22, qui est adaptée pour une administration intraveineuse.

**24.** Composition selon l'une des revendications 21 à 23, qui comprend en outre un autre anticorps thérapeutique.

**25.** Composition selon l'une des revendications 21 à 24, qui contient un dosage unitaire de 1 à 20 mg de l'anticorps ayant une affinité pour l'antigène Campath-1 ou d'un fragment de cet anticorps.

**26.** Composition selon la revendication 25, dans laquelle le dosage unitaire est 5 mg.

**27.** Anticorps selon l'une des revendications 1 à 19 ou un fragment selon la revendication 20 utilisé dans une thérapie.

**28.** Utilisation d'un anticorps selon l'une des revendications 1 à 19 ou d'un fragment selon la revendication 20 dans la préparation d'un médicament destiné à être utilisé dans une thérapie qui exige une réduction de la population des lymphocytes.

**29.** Utilisation d'un anticorps selon l'une des revendications 1 à 19 ou d'un fragment selon la revendication 20, dans la préparation d'un médicament destiné à être utilisé dans le traitement du cancer.

**30.** Utilisation d'un anticorps selon l'une des revendications 1 à 19 ou d'un fragment selon la revendication 20 dans la préparation d'un médicament destiné à être utilisé dans une immunosuppression.

27

**31.** Utilisation selon la revendication 29 ou la revendication 30, dans lequel le médicament est destiné à être utilisé dans le traitement de malignités lymphoïdes.

**32.** Utilisation selon la revendication 31, dans laquelle la malignité lymphoïde est un lymphome.

**33.** Utilisation selon la revendication 32, dans laquelle le médicament se présente sous la forme d'un dosage unitaire contenant 1 à 20 mg de l'anticorps ou du fragment de celui-ci.

**34.** Utilisation selon la revendication 33, dans laquelle le médicament est sous une forme de dosage unitaire contenant 5 mg de l'anticorps ou du fragment de celui-ci.

**35.** Utilisation selon la revendication 30, dans laquelle le médicament est destiné à être utilisé dans le traitement d'une maladie auto-immune.

**36.** Utilisation selon la revendication 30, dans laquelle le médicament est destiné à être utilisé dans le traitement d'un receveur d'un transplant.

**37.** Lignée de cellules qui expriment un anticorps ayant une affinité pour l'antigène Campath-1, ayant au moins une région déterminante de complémentarité qui est étrangère par rapport à la région constante de l'anticorps, cette(ces) région(s) déterminante(s) de complémentarité étrangère(s) étant choisie(s) parmi des régions déterminantes de complémentarité qui sont pratiquement comme identifié sur la figure 2, c'est-à-dire les résidus d'acides aminés 31 à 35, 50 à 65 et 95 à 102 de la chaîne lourde représentée sur la figure 2a, et les résidus d'acides aminés 24 à 34, 50 à 56 et 89 à 97 de la chaîne légère représentée sur la figure 2b, l'anticorps étant susceptible de se lier efficacement à l'antigène Campath-1.

**38.** Lignée de cellules selon la revendication 37, dans laquelle l'anticorps est comme défini dans l'une des revendications 2 à 20.

**39.** Lignée de cellules selon la revendication 37 ou la revendication 38, qui comprend une ou plusieurs séquences d'ADN représentées sur la figure 2 comme correspondant à une ou plusieurs de ces régions déterminantes de complémentarité.

**Revendications pour les Etats contractants suivants : ES, GR**

**1.** Procédé pour préparer un anticorps ayant une affinité pour l'antigène Campath-1, ayant au moins une région déterminante de complémentarité qui est étrangère par rapport à la région constante de l'anticorps, cette (ces) région(s) déterminante(s) de complémentarité étrangère(s) étant choisie(s) parmi des régions déterminantes de complémentarité qui sont pratiquement comme identifié sur la figure 2, c'est-à-dire des résidus d'acides aminés 31 à 35, 50 à 65 et 95 à 102 de la chaîne lourde, comme représenté sur la figure 2a, et les résidus d'acides aminés 24 à 34, 50 à 56 et 89 à 97 de la chaîne légère représentée sur la figure 2b, l'anticorps étant susceptible de se lier efficacement à l'antigène Campath-1, lequel procédé consiste à cultiver des cellules susceptibles d'exprimer l'anticorps afin d'en effectuer l'expression.

**2.** Procédé selon la revendication 1, dans lequel l'anticorps a une chaîne lourde avec au moins une région déterminante de complémentarité choisie parmi des régions déterminantes de complémentarité qui sont pratiquement comme identifié sur la figure 2a et une chaîne légère avec au moins une région déterminante de complémentarité choisie parmi des régions déterminantes de complémentarité qui sont pratiquement comme identifié sur la figure 2b.

**3.** Procédé selon la revendication 1, dans lequel l'anticorps a une chaîne lourde avec trois régions déterminantes de complémentarité qui sont pratiquement comme identifié sur la figure 2a ou une chaîne légère avec trois régions déterminantes de complémentarité qui sont pratiquement comme identifié sur la figure 2b.

**4.** Procédé pour préparer un anticorps ayant une affinité pour l'antigène Campath-1, ayant des régions déterminantes de complémentarité de chaînes lourde et légère qui sont étrangères par rapport à la

28

région constante de l'anticorps, ces régions déterminantes de complémentarité étant pratiquement comme identifié sur la figure 2, c'est-à-dire les résidus 31 à 35, 50 à 65 et 95 à 102 de la chaîne lourde représentée sur la figure 2a, et les résidus 24 à 34, 50 à 56 et 89 à 97 de la chaîne légère représentée sur la figure 2b, l'anticorps étant susceptible de se lier efficacement à l'antigène Campath-1, lequel procédé consiste à cultiver des cellules susceptibles d'exprimer l'anticorps afin d'en effectuer l'expression.

5. Procédé selon la revendication 4, dans lequel l'anticorps a des domaines variables de chaînes lourde et légère comme identifié dans les lignes inférieures de l'information de séquence de la figure 2, c'est-à-dire les résidus 1 à 113 de la chaîne lourde et les résidus 1 à 108 de la chaîne légère.

6. Procédé selon l'une des revendications 1 à 4, dans lequel les régions déterminantes de complémentarité de l'anticorps sont combinées avec des régions de cadre de domaines variables de chaînes lourde et légère qui sont d'origine humaine.

7. Procédé pour préparer un anticorps ayant une affinité pour l'antigène Campath-1, ayant des régions déterminantes de complémentarité de chaînes lourde et légère qui sont étrangères par rapport à la région constante de l'anticorps, ces régions déterminantes de complémentarité étant comme identifié sur la figure 2, c'est-à-dire les résidus 31 à 35, 50 à 65 et 95 à 102 de la chaîne lourde représentée sur la figure 2a et les résidus 24 à 34, 50 à 56 et 89 à 97 de la chaîne légère représentée sur la figure 2b, ces régions déterminantes de complémentarité étant combinées avec des régions de cadre de domaines variables de chaînes lourde et légère qui sont d'origine humaine, l'anticorps étant susceptible de se lier efficacement à l'antigène Campath-1, lequel procédé consiste à cultiver des cellules susceptibles d'exprimer l'anticorps afin d'en effectuer l'expression.

8. Procédé selon la revendication 7, dans lequel l'anticorps a des domaines variables de chaînes lourde et légère comme identifié dans les lignes supérieures de l'information de séquence de la figure 2, c'est-à-dire les résidus 1 à 113 de la chaîne lourde et les résidus 1 à 108 de la chaîne légère.

9. Procédé selon la revendication 7, dans lequel l'anticorps est comme défini dans la revendication 8, mais a une phénylalanine au résidu 27 dans la chaîne lourde à la place de sérine.

10. Procédé selon la revendication 7, dans lequel l'anticorps est comme défini dans la revendication 8, mais a une phénylalanine au résidu 27 et une thréonine au résidu 30 dans la chaîne lourde à la place de sérine.

11. Procédé selon l'une des revendications précédentes, dans lequel les domaines constants de la chaîne lourde et le domaine constant de l'anticorps sont choisis à partir d'anticorps de différents types d'espèces.

12. Procédé selon la revendication 11, dans lequel ces différents types d'espèces comprennent le rat et l'homme.

13. Procédé selon l'une des revendications 1 à 8, dans lequel les domaines constants des chaînes lourde et légère de l'anticorps sont d'origine humaine.

14. Procédé selon la revendication 13, dans lequel la région constante de l'anticorps est d'une classe IgG.

15. Procédé selon la revendication 14, dans lequel la région constante de l'anticorps est de la classe IgG1.

16. Procédé pour la préparation d'un anticorps ayant une affinité pour l'antigène Campath-1, ayant des domaines variables de chaînes lourde et légère comme identifié dans les lignes supérieures de l'information de séquence de la figure 2, c'est-à-dire les résidus 1 à 113 de la chaîne lourde et les résidus 1 à 108 de la chaîne légère, mais avec la sérine au résidu 27 dans la chaîne lourde remplacée par la phénylalanine et ayant des domaines constants de chaînes lourde et légère qui sont d'origine humaine, lequel procédé consiste à cultiver des cellules susceptibles d'exprimer l'anticorps afin d'en effectuer l'expression.

**17.** Procédé selon la revendication 16, dans lequel l'anticorps est comme défini dans la revendication 16, mais a la thréonine au résidu 30 dans la chaîne lourde à la place de sérine.

**18.** Procédé selon la revendication 16 ou la revendication 17, dans lequel la région constante de l'anticorps est d'une classe IgG.

**19.** Procédé selon la revendication 18, dans lequel la région constante de l'anticorps est de la classe IgG1.

**20.** Procédé selon l'une des revendications précédentes, dans lequel l'anticorps produit est traité pour produire un fragment qui conserve la possibilité de liaison de l'anticorps.

**21.** Procédé pour préparer une composition destinée à être administrée à des malades, contenant un anticorps comme défini dans l'une des revendications 1 à 19 ou un fragment comme défini dans la revendication 20, qui consiste à amener l'anticorps ou son fragment avec un diluant ou un porteur physiologiquement acceptable.

**22.** Procédé selon la revendication 21, dans lequel l'anticorps ou son fragment a une pureté telle qu'il est à au moins 95% en poids dépourvu d'autres matériaux biologiques.

**23.** Procédé selon la revendication 21 ou la revendication 22, dans lequel la composition est adaptée pour une administration intraveineuse.

**24.** Procédé selon l'une des revendications 21 à 23, dans lequel la composition contient en outre un autre anticorps thérapeutique.

**25.** Procédé selon l'une des revendications 21 à 24, dans lequel la composition contient un dosage unitaire de 1 à 20 mg de l'anticorps ayant une affinité pour l'antigène Campath-1 ou de son fragment.

**26.** Procédé selon la revendication 25, dans lequel le dosage unitaire est 5 mg.

**27.** Lignée de cellules qui expriment un anticorps ayant une affinité pour l'antigène Campath-1, ayant au moins une région déterminante de complémentarité qui est étrangère par rapport à la région constante de l'anticorps, cette(ces) région(s) déterminante(s) de complémentarité étrangère(s) étant choisie(s) parmi des régions déterminantes de complémentarité qui sont pratiquement comme identifié sur la figure 2, c'est-à-dire les résidus d'acides aminés 31 à 35, 50 à 65 et 95 à 102 de la chaîne lourde représentée sur la figure 2a, et les résidus d'acides aminés 24 à 34, 50 à 56 et 89 à 97 de la chaîne légère représentée sur la figure 2b, l'anticorps étant susceptible de se lier efficacement à l'antigène Campath-1.

**28.** Lignée de cellules selon la revendication 27, dans lequel l'anticorps est comme défini dans l'une des revendications 2 à 20.

**29.** Lignée de cellules selon la revendication 27 ou la revendication 28, qui comprend une ou plusieurs séquences d'ADN représenté sur la figure 2 comme correspondant à une ou plusieurs des régions déterminantes de complémentarité.

$V_H$

$V_L$

$C_H1$

$C_L$

$C_H2$

$C_H3$

| | | |
|---|---|---|
| ☐ | = | DOMAINS |
| WW | = | INTER-DOMAIN SECTIONS |
| — | = | DISULPHIDE BOND |
| V | = | VARIABLE |
| C | = | CONSTANT |
| L | = | LIGHT CHAIN |
| H | = | HEAVY CHAIN |

*Fig.1*

HindIII
5'.....↓...........ATGCAAATCCTCTGAATCTACATGGTAAATATAGGTTTGTCTATACC
■——→ RNA starts        ■——→ RNA starts
ACAAACAGAAAAACATGAGATCACAGTTCTCTCTACAGTTACTGAGCACACAGGACCTCA +60
            signal                              ↓Splice
(M  G  W  S  C  I  I  L  F  L  V  A  T  A  T)↓
CCATGGGATGGAGCTGTATCATCCTCTTCTTGGTAGCAACAGCTACAGGTAAGGGGCTCA +120
ATGAAGTTGTGGCTGAACTGGATTTTCCTTTTAACACTTTTAAAT
(M  K  L  W  L  N  W  I  F  L  L  T  L  L  N)

CAGTAGCAGGCTTGAGGTCTGGACATATATATGGGTGACAATGACATCCACTTTGCCTTT +180
Splice↓       oligos III, IV, VII
         signal      1          5            10
(G  V  H  S) Q  V  Q  L  Q  E  S  G  P  G  L  V  R
CTCTCCACAGGTGTCCACTCCCAGGTCCAACTGCAGGAGAGCGGTCCAGGTCTTGTGAGA +240
         GGTATCCAGTGTGAGGTGAAACTGTTGGAATCTGGAGGAGGCTTGGTACAG
(G  I  Q  C) E  V  K  L  L  E  S  G  G  G  L  V  Q
    15          20            25        oligo XIII        oligo X
P  S  Q  T  L  S  L  T  C  T  V  S  G  S* T  F  S* 30 CDR 1
                                                      [D  F  Y]
CCTAGCCAGACCCTGAGCCTGACCTGCACCGTGTCTGGCAGCACCTTCAGCGGATTTCTAC +300
CCGGGGGGGTTCTATGAGACTCTCCTGTGCAGGTTCTGGATTCACCTTCACTGATTTCTAC
P  G  G  S  M  R  L  S  C  A  G  S  G  F  T  F  T  [D  F  Y]

     35      oligo IX    40          45        50    52  a
[M  N] W  V  R  Q  P  P  G  R  G  L  E  W  I  G  [F  I  R  D]
ATGAACTGGGTGAGACAGCCACCTGGACGAGGTCTTGAGTGGATTGGATTTATTAGAGAC +360
ATGAACTGGATCCGCCAGCCTGCAGGGGAAGGCACCTGAGTGGCTGGGTTTTATTAGAGAC
[M  N] W  I  R  Q  P  A  G  K  A  P  E  W  L  G  [F  I  R  D]
         oligo XI
b  c 53      55    CDR 2      60          65          70
[K  A  K  G  Y  T  T  E  Y  N  P  S  V  K  G] R  V  T  M  L
AAAGCTAAAGGTTACACAACAGAGTACAATCCATCTGTGAAGGGGGAGAGTGACAATGCTG +420
AAAGCTAAAGGTTACACAACAGAGTACAATCCATCTGTGAAGGGGCGGTTCACCATCTCC
[K  A  K  G  Y  T  T  E  Y  N  P  S  V  K  G] R  F  T  I  S

        75          80    82  a  b  c 83    85
V  D  T  S  K  N  Q  F  S  L  R  L  S  S  V  T  A  A  D  T
GTAGACACCAGCAAGAACCAGTTCAGCCTGAGACTCAGCAGCGTGACAGCCGCCGACACC +480
AGAGATAATACCCAAAACATGCTCTATCTTCAAATGAACACCCTAAGAGCTGAGGACACT
R  D  N  T  Q  N  M  L  Y  L  Q  M  N  T  L  R  A  E  D  T
                              oligo XII
     90          95  CDR 3     100  a  b 101      105
A  V  Y  Y  C  A  R  [E  G  H  T  A  A  P  F  D  Y] W  G  Q
GCGGTCTATTATTGTGCAAGAGAGGGCCACACTGCTGCTCCTTTTGATTACTGGGGTCAA +540
GCCACTTACTACTGTGCAAGAGAGGGCCACACTGCTGCTCCTTTTGATTACTGGGGCCAA
A  T  Y  Y  C  A  R  [E  G  H  T  A  A  P  F  D  Y] W  G  Q
    oligos V, VI, VII
        110      113  ↓Splice
G  S  L  V  T  V  S  S  ↓                          ↓BamHI
GGCAGCCTCGTCACAGTCTCCTCAGGT.........................↘...3' +600
GGAGTCATGGTCACAGTCTCCTCA
G  V  M  V  T  V  S  S

Oligonucleotides: I: 5'-GGC CAG TGG ATA GAC-3', III: 5'-CAG TTT CAT CTA
GAA CTG GAT A-3', IV: 5'-GCA GTT GGG TCT AGA AGT GGA CAC C-3',
V: 5'-TCA GCT GAG TCG ACT GTG AC-3', VI: 5'-TCA CCT GAG TCG ACT GTG
AC-3', VII: 5'-AGT TTC ACC TCG AGT GGA CAC CT-3', VIII: 5'-TCA CCT GAG
GAG ACT GTG AC-3'; IX: 5'-GGC TGG CGA ATC CAG TT-3', X: 5'-CTG TCT CAC
CCA GTT CAT GTA GAA ATC GCT GAA GGT GCT-3', XI: 5'-CAT TGT CAC TCT
CCC CTT CAC AGA TGG ATT GTA CTC TGT TGT GTA ACC TTT AGC TTT GTC
TCT AAT AAA TCC AAT CCA CTC-3', XII: 5'-GCC TTG ACC CCA GTA ATC AAA
AGG AGC AGC AGT GTG GCC CTC TCT TGC ACA ATA-3', XIII: 5'-AGA AAT
CGG/C TGA AGG TGA AGC CAG ACA C-3'.

*Fig. 2a*

HindIII|
5'.....↓.......ATGCAAATCCTCTGAATCTACATGGTAAATATAGGTTTGTCTATACC
■——→RNA starts        ■——→RNA starts
ACAAACAGAAAAACATGAGATCACAGTTCTCTCTACAGTTACTGAGCACACAGGACCTCA +60
                                                          ATGA
                                                           M

            signal                                    |Splice
( M  G  W  S  C  I  I  L  F  L  V  A  T  A  T )↓
CCATGGGATGGAGCTGTATCATCCTCTTCTTGGTAGCAACAGCTACAGGTAAGGGGCTCA +120
TGGCTGCACTTCAACTCTTAGGGGTAGCTGCTAGCTCTGGCTCCCAG
( M  A  A  L  Q  L  L  L  G  V  A  A  S  S  G  S  Q )

CAGTAGCAGGCTTGAGGTCTGGACATATATATGGGTGACAATGACATCCACTTTGCCTTT +180

    Splice↓ signal         1          5          10
( G  V  H  S ) D  I  Q  M  T  Q  S  P  S  S  L  S  A
CTCTCCACAGGTGTCCACTCCGACATCCAGATGACCCAGAGCCCAAGCAGCCTGAGCGCC +240
        GCCATGAGATGTGACATCAAGATGACCCAGTCTCCCTCATTCCTGTCTGCA
( A  M  R  C ) D  I  K  M  T  Q  S  P  S  F  L  S  A

   15              20              25      oligo XIV  30      CDR 1
   S  V  G  D  R  V  T  I  T  C [ K  A  S  Q  N  I  D  K  Y  L
AGCGTGGGTGACAGAGTGACCATCACCTGTAAAGCAAGTCAGAATATTGACAAATACTTA +300
TCTGTGGGAGACAGAGTCACTCTCAACTGCAAAGCAAGTCAGAATATTGACAAATACTTA
   S  V  G  D  R  V  T  L  N  C [ K  A  S  Q  N  I  D  K  Y  L

   35            40              45       oligo XV  50      CDR 2
[N] W  Y  Q  Q  K  P  G  K  A  P  K  L  L  I  Y [N  T  N  N
AACTGGTACCAGCAGAAGCCAGGTAAGGCTCCAAAGCTGCTGATCTACAATACAAACAAT +360
AACTGGTATCAGCAAAAGCTTGGAGAATCTCCCAAACTCCTGATATATAATACAAACAAT
[N] W  Y  Q  Q  K  L  G  E  S  P  K  L  L  I  Y [N  T  N  N

   55            60              65              70
[L  Q  T] G  V  P  S  R  F  S  G  S  G  S  G  T  D  F  T  F
TTGCAAACGGGTGTGCCAAGCAGATTCAGCGGTAGCGGTAGCGGTACCGACTTCACCTTC +420
TTGCAAACGGGCATCCCATCAAGGTTCAGTGGCAGTGGATCTGGTACTGATTTCACACTC
[L  Q  T] G  I  P  S  R  F  S  G  S  G  S  G  T  D  F  T  L

   75              80              85       oligo XVI  90    CDR 3
   T  I  S  S  L  Q  P  E  D  I  A  T  Y  V  C [L  Q  H  I  S
ACCATCAGCAGCCTCCAGCCAGAGGACATCGCCACCTACTACTGCTTGCAGCATATAAGT +480
ACCATCAGCAGCCTGCAGCCTGAAGATGTTGCCACATATTTCTGCTTGCAGCATATAAGT
   T  I  S  S  L  Q  P  E  D  V  A  T  Y  F  C [L  Q  H  I  S

   95          100           105         108
[R  P  R  T] F  G  Q  G  T  K  V  E  I  K  R
AGGCCGCGCACGTTCGGCCAAGGGACCAAGGTGGAAATCAAACGTGAGTAGAATTTAAAC +540
AGGCCGCGCACGTTTGGAACTGGGACCAAGCTGGAGCTGAAACGG
[R  P  R  T] F  G  T  G  T  K  L  E  L  K  R
              BamHI
TTTGCTTCCTCAGTTGGATCC-3'


Oligonucleotides: II: 5'-TGC AGC ATC AGC C-3', XIV: 5'-CTG CTG GTA CCA
GTT TAA GTA TTT GTC AAT ATT CTG ACT TGC TTT ACA GGT GAT GGT-3',
XV: 5'-GCT TGG CAC ACC CGT TTG CAA ATT GTT TGT ATT GTA GAT CAG
CAG-3', XVI: 5'-CCC TTG GCC GAA CGT GCG CGG CCT ACT TAT ATG CTG CAA
GCA GTA GTA GGT-3'.

*Fig.2b*

Sequence of the synthetic gene HUVLLYSO

```
            D   I   Q   M   T   Q   S   P   S   S   L   S   A   S   V   G   D   R   V
CTGCA  GACATCCAGATGACCCAGAGCCCAAGCAGCCTGAGCGCCAGCGTGGGTGACAGAG
GACGT  CTGTAGGTCTACTGGGTCTCGGGTTCGTCGGACTCGCGGTCGCACCCACTGTCTC
         10          20          30          40          50          60


            T   I   T   C   R   A   S   G   N   I   H   N   Y   L   A   W   Y   Q   Q   K
TGACCATCACCTGTAGAGCCAGCGGTAACATCCACAACTACCTGGCTTGGTACCAGCAGA
ACTGGTAGTGGACATCTCGGTCGCCATTGTAGGTGTTGATGGACCGAACCATGGTCGTCT
           70          80          90         100         110         120


            P   G   K   A   P   K   L   L   I   Y   Y   T   T   T   L   A   D   G   V   P
AGCCAGGTAAGGCTCCAAAGCTGCTGATCTACTACACCACCACCCTGGCTGACGGTGTGC
TCGGTCCATTCCGAGGTTTCGACGACTAGATGATGTGGTGGTGGGACCGACTGCCACACG
          130         140         150         160         170         180


            S   R   F   S   G   S   G   S   G   T   D   F   T   F   T   I   S   S   L   Q
CAAGCAGATTCAGCGGTAGCGGTAGCGGTACCGACTTCACCTTCACCATCAGCAGCCTCC
GTTCGTCTAAGTCGCCATCGCCATCGCCATGGCTGAAGTGGAAGTGGTAGTCGTCGGAGG
          190         200         210         220         230         240


            P   E   D   I   A   T   Y   Y   C   Q   H   F   W   S   T   P   R   T   F   G
AGCCAGAGGACATCGCCACCTACTACTGCCAGCACTTCTGGAGCACCCCAAGGACGTTCG
TCGGTCTCCTGTAGCGGTGGATGATGACGGTCGTGAAGACCTCGTGGGGTTCCTGCAAGC
          250         260         270         280         290         300


            Q   G   T   K   V   E   I   K   R
GCCAAGGGACCAAGGTGGAAATCAAACGTGAGTAGAATTTAAACTTTGCTTCCTCAGTTG
CGGTTCCCTGGTTCCACCTTTAGTTTGCACTCATCTTAAATTTGAAACGAAGGAGTCAAC
          310         320         330         340         350         360


GATCCTAGAATTC
CTAGGATCTTAAG
          370
```

*Fig.3*

34

```
                                               ATGCAAATCCTCTGAAT
                              •                            •
CTACATGGTAAATATAGGTTTGTCTATACCACAAACAGAAAAACATGAGATCACAGTTCT

                                       M   G   W   S   C   I   I   L   F
CTCTACAGTTACTGAGCACACAGGACCTCACCATGGGATGGAGCTGTATCATCCTCTTCT

  L   V   A   T   A   T
TGGTAGCAACAGCTACAGGTAAGGGGCTCACAGTAGCAGGCTTGAGGTCTGGACATATAT

                                                         1
                                    G   V   H   S   D   I   Q
ATGGGTGACAATGACATCCACTTTGCCTTTCTCTCCACAGGTGTCCACTCCGACATCCAG

     5               10              15              20
  M   T   Q   S   P   S   S   L   S   A   S   V   G   D   R   V   T   I   T   C
ATGACCCAGAGCCCAAGCAGCCTGAGCGCCAGCGTGGGTGACAGAGTGACCATCACCTGT

  ****************************
     25              30              35              40
  R   A   S   G   N   I   H   N   Y   L   A   W   Y   Q   Q   K   P   G   K   A
AGAGCCAGCGGTAACATCCACAACTACCTGGCTTGGTACCAGCAGAAGCCAGGTAAGGCT

                    ******************
     45              50              55              60
  P   K   L   L   I   Y   Y   T   T   T   L   A   D   G   V   P   S   R   F   S
CCAAAGCTGCTGATCTACTACACCACCACCCTGGCTGACGGTGTGCCAAGCAGATTCAGC

     65              70              75              80
  G   S   G   S   G   T   D   F   T   F   T   I   S   S   L   Q   P   E   D   I
GGTAGCGGTAGCGGTACCGACTTCACCTTCACCATCAGCAGCCTCCAGCCAGAGGACATC

                    ********************
     85              90              95              100
  A   T   Y   Y   C   Q   H   F   W   S   T   P   R   T   F   G   Q   G   T   K
GCCACCTACTACTGCCAGCACTTCTGGAGCACCCCAAGGACGTTCGGCCAAGGGACCAAG

   105       108
  V   E   I   K   R
GTGGAAATCAAACGTGAGTAGAATTTAAACTTTGCTTCCTCAGTTGGATCCTAGAATTC
```

*Fig.4*

HuVHCAMP-RaIgG2b

HuVHCAMP-HuIgG1
HuVLCAMP-HuIgκ

1

2

RaVHCAMP-HuIgG1,2,3,4

3

rat

human

Fig.5

Fig.6

a.)

b.)

Fig.7

a.)

b)

Fig.8

Fig.9

Fig.10

*Fig.11*

Fig.12

Days since start of antibody therapy